(19) Europäisches Patentamt
European Patent Office
Office européen des brevets

(11) Veröffentlichungsnummer : **0 049 486**
**B1**

# (12) EUROPÄISCHE PATENTSCHRIFT

(45) Veröffentlichungstag der Patentschrift : **18.09.85**

(51) Int. Cl.⁴ : **A 61 K 31/28**, A 61 K 31/32, C 07 C 49/92, C 07 C 45/77

(21) Anmeldenummer : **81107815.3**

(22) Anmeldetag : **01.10.81**

(54) **Metallkomplexe zur Anwendung bei der Behandlung von Krebskrankheiten.**

(30) Priorität : **04.10.80 DE 3037665**
**19.12.80 DE 3048109**
**22.04.81 DE 3115919**
**02.09.81 DE 3134711**

(43) Veröffentlichungstag der Anmeldung : **14.04.82 Patentblatt 82/15**

(45) Bekanntmachung des Hinweises auf die Patenterteilung : **18.09.85 Patentblatt 85/38**

(84) Benannte Vertragsstaaten : **AT BE CH DE FR GB IT LI LU NL SE**

(56) Entgegenhaltungen :
**DE-A- 2 801 355**
**DE-A- 2 923 334**
**FR-A- 1 341 731**
**BULLETIN OF THE ACADEMY OF SCIENCES OF THE USSR, Band 24, Nr. 7, Juli 1975, Seiten 1482-1485 New York, U.S.A. E.M. BRAININA et al.: "Reaction of chelate cyclopentadienyl compounds of zirconium and hafnium with bromine"**
**JOURNAL OF INORGANIC & NUCLEAR CHEMISTRY, Band 38, 1976, Seiten 389-391 Pergamon Press Oxford, G.B. B.P. BACHLAS et al.: "Some new ion-pair complexes of tin(IV) and dihalotin bis(beta-diketonates)**
**CHEMICAL ABSTRACTS, Band 55, nr. 4, 20. Februar 1961, Spalte 3840b GLORIA E. ZANDER-PRINCIPATI et al.: "Effect of zirconium citrate on the latent period of Sr89-induced bone cancer in mice"**

(73) Patentinhaber : **Byk Gulden Lomberg Chemische Fabrik GmbH**
**Byk-Gulden-Strasse 2**
**D-7750 Konstanz (DE)**

(72) Erfinder : **Keller, Heimo J., Prof. Dr.**
**Angelweg 28**
**D-6900 Heidelberg (DE)**
Erfinder : **Keppler, Bernhard, Dr.**
**Luisenstrasse 35**
**D-6832 Hockenheim (DE)**
Erfinder : **Krüger, Uwe, Dr.**
**Neuhauser Strasse 11**
**D-7750 Konstanz (DE)**
Erfinder : **Linder, Rudolf, Dr.**
**Hermann-von-Vincaristrasse 41**
**D-7750 Konstanz (DE)**

**EP 0 049 486 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Technisches Gebiet

Die Erfindung bezieht sich auf Metallkomplexe zur Anwendung bei der Behandlung von Krebskrankheiten.

Stand der Technik

Kürzlich wurde ein die Komplexverbindung cis-Diammindichloroplatin (II) enthaltendes Arzneimittel als Chemotherapeutikum gegen Krebs in den Handel gebracht. Diese unter dem International Nonproprietary Name (INN) Cisplatin bekannte Verbindung hat sich als äußerst potentes Antitumormittel, insbesondere bei der Behandlung von Hodentumoren, aber auch z. B. von Eierstocktumoren und kleinzelligen Bronchialkarzinomen erwiesen. Nachteilig an Cisplatin ist seine relativ große Toxizität. Besonders gravierend sind seine Nephrotoxizität, sowie seine zu bleibenden Gehörschäden führende Wirkung. Nieren- und Gehörschäden werden bereits nach Verabreichung einer einzigen therapeutischen Dosis mit erheblicher Häufigkeit festgestellt. Neben der nephro- und haematotoxischen Wirkung sind für die Patienten vor allem noch die langanhaltende starke Übelkeit und der damit verbundene Brechreiz äußerst unangenehm.

Es wurden in letzter Zeit zahlreiche andere Platinkomplexe (DE-OS 24 45 418, DE-OS 28 37 237, DE-OS 26 26 559, DE-OS 25 39 179) und Komplexverbindungen anderer Übergangsmetalle als cytostatisch wirkende Mittel vorgeschlagen. In der DE-OS 28 01 355 werden einem braunen amorphen Komplex, gewonnen durch Reaktion von Ascorbinsäure mit einer Titan (III)- und einer Kupfer (II) verbindung (Molverhältnis 36 : 1 : 6) kurative und prophylaktische Wirkungen gegen u. a. Leukämie zugeschrieben. Es wurde berichtet, daß Titanocen-, Zirconocen- und Hafnocendichlorid hemmend gegenüber dem Ehrlich-Ascites-Tumor der Maus wirken [P. Köpf-Maier, B. Hesse, H. Köpf, J. Cancer Res. Clin. Oncol. *96*, 43 (1980)]. Von Dialkylzinndihalogen-phenanthrolin- bzw. -bipyridyl-Komplexverbindungen wurden inhibierende Wirkungen auf die P388 Leukämie bei Mäusen angegeben (A. J. Crowe u. P. J. Smith, Chemistry and Industry, 1. März 1980, S. 200).

Bei E. M. Brainina et al. Bull. Acad. Sci. USSR *23*, 1482-85 (1975) wird die Herstellung von Bis- und Tris(dibenzoylmethanato) zirkonium (IV) bromid und -hafnium (IV) bromid angegeben. B. P. Backlas et al. Journ. Inorg. Nucl. Chem. *38*, 389-391 (1976) beschreiben die Umsetzung von Zinntetrachlorid mit p-Fluorobenzoylaceton.

Darstellung der Erfindung

Überraschend wurde nun gefunden, daß die Komplexverbindungen der allgemeinen Formel I

$$[R^1(CH_2)_mC(O)CR^3C(O)R^2]_{2+n}MX_{2-n} \qquad (I)$$

worin

M Zinn, Titan, Zirkon oder Hafnium,

$R^1$ Wasserstoff oder einen Phenylrest, wobei der Phenylrest durch Fluor, Chlor, Brom, Nitro, $C_1$-$C_4$-Alkyl, $C_1$-$C_4$-Alkoxy oder Trifluormethyl einfach oder mehrfach substituiert sein kann,

$R^2$ Methyl oder $R^1$ mit Ausnahme von Wasserstoff,

$R^3$ Wasserstoff oder Chlor,

X Fluor, Chlor oder Brom, jedoch nicht Fluor wenn M die Bedeutung Zirkon oder Hafnium hat,

m die Zahl 1 oder 0 oder, falls $R^1$ die Bedeutung Wasserstoff hat, die Zahl 1, und

n die Zahl 0 oder, falls M die Bedeutung Zirkon oder Hafnium hat, die Zahl 1 oder 0, bedeuten,

eine dem Cisplatin vergleichbare cytostatische Wirksamkeit aufweisen, aber weitaus ungiftiger als dieses sind. Diese Verbindungen besitzen demnach im Vergleich zu Cisplatin eine bedeutend größere therapeutische Breite. Sie eignen sich als Chemotherapeutikum zur Behandlung von Krebskrankheiten.

Gegenstand der Erfindung sind daher Komplexverbindungen der allgemeinen Formel I,

$$[R^1(CH_2)_mC(O)CR^3C(O)R^2]_{2+n}MX_{2-n} \qquad (I)$$

worin

M Zinn, Titan, Zirkon oder Hafnium,

$R^1$ Wasserstoff oder einen Phenylrest, wobei der Phenylrest durch Fluor, Chlor, Brom, Nitro, $C_1$-$C_4$-Alkyl, $C_1$-$C_4$-Alkoxy oder Trifluormethyl einfach oder mehrfach substituiert sein kann,

$R^2$ Methyl oder $R^1$ mit Ausnahme von Wasserstoff,

$R^3$ Wasserstoff oder Chlor,

X Fluor, Chlor oder Brom, jedoch nicht Fluor wenn M die Bedeutung Zirkon oder Hafnium hat,

m die Zahl 1 oder 0 oder, falls $R^1$ die Bedeutung Wasserstoff hat, die Zahl 1, und

n die Zahl 0 oder, falls M die Bedeutung Zirkon oder Hafnium hat, die Zahl 1 oder 0, bedeuten, zur Anwendung bei der Behandlung von Krebskrankheiten.

Bevorzugt sind Komplexverbindungen der allgemeinen Formel I'

$$[R^{1'}(CH_2)_{m'}C(O)CR^{3'}C(O)R^{2'}]_{2+n'}M'X'_{2-n'} \qquad (I'),$$

worin

M' Zinn, Titan, Zirkon oder Hafnium,

$R^{1'}$ einen Phenylrest, der 4-Chlor, 4-Brom-, 3-Nitro, 4-Methoxy-, 3,4-Dimethoxy-, 4-Methyl- oder 2,4,6-Trimethyl-substituiert sein kann,

$R^{2'}$ Methyl oder $R^{1'}$,

$R^{3'}$ Wasserstoff oder Chlor,

X' Fluor, Chlor oder Brom, jedoch nicht Fluor, wenn M' die Bedeutung Zirkon oder Hafnium hat,

m' die Zahl 1 oder 0 und

n' die Zahl 0 oder, falls M' die Bedeutung Zirkon oder Hafnium hat, die Zahl 1 oder 0, bedeuten, zur Anwendung bei der Behandlung von Krebskrankheiten.

Besonders bevorzugt sind Komplexverbindungen der allgemeinen Formel I''

$$[R^{1''}C(O)CR^{3''}C(O)R^{2''}]_{2+n''}M''X''_{2-n''} \qquad (I'')$$

worin

M'' Zinn, Titan, Zirkon oder Hafnium,

$R^{1''}$ einen Phenylrest,

$R^{2''}$ einen Methylrest,

$R^{3''}$ Wasserstoff oder Chlor,

X'' Fluor, Chlor oder Brom, jedoch nicht Fluor wenn M'' die Bedeutung Zirkon oder Hafnium hat, und

n'' die Zahl 0 oder, falls M'' die Bedeutung Zirkon oder Hafnium hat, die Zahl 1 oder 0, bedeuten, zur Anwendung bei der Behandlung von Krebskrankheiten.

Besondere Ausgestaltungen der Erfindung sind Verbindungen der vorstehenden Formeln I, I' bzw. I'', worin die Variablen M, M' bzw. M'', $R^1$, $R^{1'}$ bzw, $R^{1''}$, $R^2$, $R^{2'}$ bzw. $R^{2''}$, $R^3$, $R^{3'}$ bzw. $R^{3''}$, n, n' bzw. n'' und m bzw. m' die vorstehend angegebenen Bedeutungen haben, X, X' bzw. X'' jedoch jeweils nur Chlor bedeuten, zur Anwendung bei der Behandlung von Krebskrankheiten.

Weitere besondere Ausgestaltungen der Erfindung sind Verbindungen der vorstehenden, Formeln I, I' bzw. I'', worin die Variablen $R^1$, $R^{1'}$ bzw. $R^{1''}$, $R^2$, $R^{2'}$ bzw. $R^{2''}$, $R^3$, $R^{3'}$ bzw. $R^{3''}$, X, X' bzw. X'', n, n' bzw. n'' und m bzw. m' die vorstehend angegebenen Bedeutungen haben, M, M' bzw. M'' jedoch jeweils nur die Bedeutung Titan, Zirkon oder Hafnium, vorzugsweise Titan, haben, zur Anwendung bei der Behandlung von Krebskrankheiten.

Besonders bevorzugt sind die Verbindungen Dichlorobis(benzoylacetonato) titan (IV), Dichlorobis(benzoylacetonato) zirkon (IV) und/oder Dichlorobis(benzoylacetonato) hafnium (IV) zur Anwendung bei der Behandlung von Krebskrankheiten.

Ein weiterer Gegenstand der Erfindung ist die Verwendung der Verbindungen der allgemeinen Formel I, I' bzw. I'' zur Herstellung von Arzneimitteln gegen Krebskrankheiten, auf nichtchemischem Weg.

Die Verbindungen der allgemeinen Formel $I^a$

$$[R^{1a}(CH_2)_{m^a}C(O)CR^{3a}C(O)R^{2a}]_{2+n^a}M^aX^a_{2-n^a} \qquad (I^a),$$

worin

$M^a$ Zinn, Titan, Zirkon, Hafnium,

$R^{1a}$ einen durch Fluor, Chlor, Brom, Nitro, $C_1$-$C_4$-Alkyl, $C_1$-$C_4$-Alkoxy oder Trifluormethyl einfach oder mehrfach substituierten Phenylrest, und falls $M^a$ Zirkon und Hafnium darstellt zusätzlich einen Phenylrest,

$R^{2a}$ Methyl oder $R^{1a}$ einschließlich Phenyl für alle Bedeutungen von $M^a$,

$R^{3a}$ Wasserstoff oder Chlor,

$X^a$ Fluor, Chlor oder Brom,

jedoch nicht Fluor, wenn $M^a$ für Zirkon oder Hafnium, und nicht Chlor, wenn $M^a$ für Titan oder Zinn und $R^{1a}$ für p-Fluor-, p-Chlor- oder p-Bromphenyl und $R^{2a}$ für Methyl oder wenn $M^a$ für Zirkon und $R^{1a}$ für Phenyl oder wenn $M^a$ für Hafnium und $R^{1a}$ für Phenyl und $R^{2a}$ für Methyl und $n^a$ für 1 oder wenn $M^a$ für Sn und $R^{1a}$ und $R^{2a}$ für Phenyl, und nicht Brom, wenn $M^a$ für Zirkon oder Hafnium $R^{1a}$ und $R^{2a}$ für Phenyl, oder wenn $M^a$ für Zinn und $R^{1a}$ für Phenyl oder p-Fluorphenyl, und $R^{2a}$ für Methyl stehen.

$m^a$ die Zahl 1 oder 0, und

$n^a$ die Zahl 0 oder, falls $M^a$ die Bedeutung Zirkon oder Hafnium hat, die Zahl 1 oder 0, bedeuten, sind neu und daherein weiterer Gegenstand der Erfindung.

Bevorzugt sind Verbindungen der allgemeinen Formel $I^{a'}$

$$[R^{1a'}(CH_2)_{m^{a'}}C(O)CR^{3a'}C(O)R^{2a'}]_{2+n^{a'}}M^{a'}X^{a'}_{2-n^{a'}} \quad (I^{a'}),$$

worin

$M^{a'}$ Zirkon oder Hafnium,

$R^{1a'}$ Phenyl,

$R^{2a'}$ Methyl,

$R^{3a'}$ Wasserstoff oder Chlor,

$X^{a'}$ Chlor oder Brom, aber nicht Chlor, falls $M^{a'}$ für Zirkon oder falls $M^{a'}$ für Hafnium und $n^{a'}$ für die Zahl 1 stehen,

$m^{a'}$ die Zahl 1 oder 0 und

$n^{a'}$ die Zahl 1 oder 0 bedeuten.

Besonders bevorzugt sind Dichlorobis(benzoylacetonato) hafnium (IV), Dibromobis(benzoylacetonato) zirkon (IV) und Dibromobis(benzoylacetonato) hafnium (IV).

Gegenstand der Erfindung ist weiterhin ein Verfahren zur Herstellung der Verbindungen der allgemeinen Formeln $I^a$ bzw. $I^{a'}$, worin $R^{1a}$ bzw. $R^{1a'}$, $R^{2a}$ bzw. $R^{2a'}$, $R^{3a}$ bzw. $R^{3a'}$, $X^a$ bzw. $X^{a'}$, $m^a$ bzw. $m^{a'}$ und $n^a$ bzw. $n^{a'}$ die vorstehend angegebenen Bedeutungen haben, das dadurch gekennzeichnet ist, daß man ein Metalltetrahalogenid $M^aX_4^a$ bzw. $M^{a'}X_4^{a'}$, wobei $M^a$ bzw. $M^{a'}$ und $X^a$ bzw. $X^{a'}$ die vorstehend angegebenen Bedeutungen haben, unter Feuchtigkeitsausschluß in einem inerten Lösungsmittel mit einem Diketon $R^{1a}(CH_2)_{m^a}C(O)CHR^{3a}C(O)R^{2a}$ bzw. $R^{1a'}(CH_2)_{m^{a'}}C(O)CHR^{3a'}C(O)R^{2a'}$, wobei $R^{1a}$ bzw. $R^{1a'}$, $R^{2a}$ bzw. $R^{2a'}$, $R^{3a}$ bzw. $R^{3a'}$ und $m^a$ bzw. $m^{a'}$ die vorstehend angegebenen Bedeutungen haben, im molaren Verhältnis von höchstens 1 : 2 umsetzt.

Die Komplexverbindungen werden als Arzneimittel vor allem intravenös, aber auch intramuskulär, intraperitoneal, subkutan, rektal oder peroral verabreicht. Auch eine äußerliche Applikation ist möglich. Bevorzugt ist die Verabreichung durch intravenöse Injektion oder intravenöse Infusion.

Die Arzneimittel werden nach an sich bekannten Verfahren hergestellt, wobei die Komplexverbindung als solche oder gegebenenfalls in Kombination mit geeigneten pharmazeutischen Trägerstoffen eingesetzt wird. Enthalten die neuen pharmazeutischen Zubereitungen neben dem Wirkstoff pharmazeutische Trägerstoffe, beträgt der Wirkstoffgehalt dieser Mischungen 0,1 bis 99,5, vorzugsweise 0,5 bis 95 Gewichtsprozent der Gesamtmischung.

In Übereinstimmung mit der Erfindung wird der Wirkstoff in jeder geeigneten Formulierung angewandt unter der Voraussetzung, daß die Ausbildung bzw. Aufrechterhaltung von ausreichenden Wirkstoffspiegeln gewährleistet ist. Das kann beispielsweise durch orale oder parenterale Gabe in geeigneten Dosen erreicht werden. Vorteilhafterweise liegt die pharmazeutische Zubereitung des Wirkstoffs in Form von Einheitsdosen vor, die auf die gewünschte Verabreichung abgestimmt sind. Eine Einheitsdosis kann zum Beispiel eine Tablette, ein Dragee, eine Kapsel, ein Suppositorium oder eine gemessene Volumenmenge eines Pulvers, eines Granulates, einer Lösung, einer Emulsion oder einer Suspension sein.

Unter « Einheitsdosis » im Sinne der vorliegenden Erfindung wird eine physikalisch bestimmte Einheit, die eine individuelle Menge des aktiven Bestandteils in Kombination mit einem pharmazeutischen Trägerstoff enthält, verstanden, deren Wirkstoffgehalt einem Bruchteil oder einem Vielfachen einer therapeutischen Einzeldosis entspricht. Eine Einzeldosis enthält vorzugsweise die Menge Wirkstoff, die bei einer Applikation verabreicht wird und die gewöhnlich einer ganzen, einer halben, einer drittel oder einer viertel Tagesdosis entspricht. Wenn für eine einzelne therapeutische Verabreichung nur ein Bruchteil, wie die Hälfte oder ein Viertel, der Einheitsdosis benötigt wird, ist die Einheitsdosis vorteilhafterweise teilbar, z. B. in Form einer Tablette mit Bruchkerbe.

Die pharmazeutischen Zubereitungen gemäß der Erfindung können, wenn sie in Einheitsdosen vorliegen und für die Applikation z. B. am Menschen bebestimmt sind, etwa 0,1 bis 500 mg, vorteilhafterweise 10 bis 200 mg und insbesondere 50 bis 150 mg Wirkstoff enthalten.

Im allgemeinen erweist es sich in der Humanmedizin als vorteilhaft, den oder die Wirkstoffe bei parenteraler Gabe in einer Tagesdosis von etwa 0,1 bis etwa 5, vorzugsweise 1 bis 3 mg/kg Körpergewicht, gegebenenfalls in Form mehrerer, vorzugsweise 1 bis 3 Einzelgaben zur Erzielung der gewünschten Ergebnisse zu verabreichen. Eine Einzelgabe enthält den oder die Wirkstoffe in Mengen von etwa 0,1 bis etwa 5, vorzugsweise 0,5 bis 3 mg/kg Körpergewicht. Bei einer oralen Behandlung können ähnliche Dosierungen zur Anwendung kommen.

Die therapeutische Verabreichung der pharmazeutischen Zubereitung kann 1 bis 4 mal am Tage zu festgelegten oder variierenden Zeitpunkten erfolgen, z. B. jeweils nach den Mahlzeiten und/oder am Abend. Es kann jedoch erforderlich sein, von den genannten Dosierungen abzuweichen, und zwar in Abhängigkeit von der Art, dem Körpergewicht und dem Alter des zu behandelnden Individuums, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Wie bei der internistischen Tumortherapie üblich, kann zur Reduzierung des Nebenwirkungsrisikos die Behandlung mit den erfindungsgemäßen Arzneimitteln kombiniert werden mit der Verabreichung anderer Cytostatica mit

unterschiedlichen Wirkungsspektren. Es kann auch zweckmäßig sein, die Behandlung nach dem Prinzip der zyklischen Cytostaticatherapie durchzuführen. Hierbei wird nach jeder Behandlung eine Erholungsphase eingelegt. Man macht sich dabei die Erfahrung zunutze, daß gesundes Gewebe der meisten Organe schneller regeneriert als malignes Gewebe.

Die Festlegung der jeweils erforderlichen optimalen Dosierung und Applikationsart der Wirkstoffe kann durch jeden Fachmann aufgrund seines Fachwissens leicht erfolgen.

Die pharmazeutischen Zubereitungen bestehen in der Regel aus den Komplexverbindungen und nichttoxischen, pharmazeutisch verträglichen Arzneimittelträgern, die als Zumischung oder Verdünnungsmittel in fester, halbfester oder flüssiger Form oder als Umhüllungsmittel, beispielsweise in Form einer Kapsel, eines Tablettenüberzugs, eines Beutels oder eines anderen Behältnisses, für den therapeutisch aktiven Bestandteil in Anwendung kommen. Ein Trägerstoff kann z. B. als Vermittler für die Arzneimittelaufnahme durch den Körper, als Formulierungshilfsmittel, als Süßungsmittel, als Geschmackskorrigens, als Farbstoff oder als Konservierungsmittel dienen.

Zur oralen Anwendung können z. B. Tabletten, Dragees, harte und weiche Kapseln, z. B. aus Gelatine, dispergierbare Pulver, Granulate, wäßrige und ölige Suspensionen, Emulsionen, Lösungen oder Sirupe kommen.

Tabletten können inerte Vedünnungsmittel, z. B. Calciumcarbonat, Calciumphosphat, Natriumphosphat oder Lactose ; Granulierungs- und Verteilungsmittel, z. B. Maisstärke oder Alginate ; Bindemittel, z. B. Stärke, Gelatine oder Akaziengummi ; und Gleitmittel, z. B. Aluminium- oder Magnesiumstearat, Talkum oder Silikonöl, enthalten. Sie können zusätzlich mit einem Überzug versehen sein, der auch so beschaffen sein kann, daß er eine verzögerte Auflösung und Resorption des Arzneimittels im Gastrointestinaltrakt bewirkt, so daß z. B. eine bessere Verträglichkeit, Protrahierung oder eine Retardierung erreicht wird. Gelatinekapseln können den Arzneistoff vermischt mit einem festen z. B. Calciumcarbonat oder Kaolin, oder einem öligen z. B. Oliven-, Erdnuß- oder Paraffinöl, Verdünnungsmittel enthalten.

Wäßrige Suspensionen können Suspendiermittel, z. B. Natriumcarboxymethylcellulose, Methylcellulose, Hydroxypropylcellulose, Natriumalginat, Polyvinylpyrrolidon, Traganthgummi oder Akaziengummi ; Dispergier- und Benetzungsmittel, z. B. Polyoxyethylenstearat, Heptadecaethylenoxycetanol, Polyoxyethylensorbitanfettsäureester wie z. B. Polyoxyethylensorbitanmonooleat, sowie Lecithin ; Konservierungsmittel, z. B. Methyl- oder Propylhydroxybenzoate ; Geschmacksmittel ; Süßungsmittel, z. B. Saccharose, Lactose, Natriumcyclamat, Dextrose, Invertzuckersirup, enthalten.

Ölige Suspensionen können z. B. Erdnuß-, Oliven-, Sesam-, Kokos- oder Paraffinöl und Verdickungsmittel, wie z. B. Bienenwachs, Hartparaffin, oder Cetylalkohol, enthalten ; ferner Süßungsmittel, Geschmacksmittel und Antioxidantien.

In Wasser dispergierbare Pulver und Granulate können die Komplexverbindungen in Mischung mit Dispergier-, Benetzungs- und Suspendiermitteln, z. B. den oben genannten, sowie Süßungsmitteln, Geschmacksmitteln und Farbstoffen enthalten.

Emulsionen können z. B. Oliven-, Erdnuß- oder Paraffinöl neben Emulgiermitteln, wie z. B. Akaziengummi, Traganthgummi, Phosphatiden, Sorbitanmonooleat, Polyoxyethylensorbitanmonooleat, und Süßungs- und Geschmacksmittel enthalten.

Zur parenteralen Anwendung des Arzneistoffes dienen steril injizierbare wäßrige Suspensionen, isotonische Salzlösungen oder sonstige Lösungen, die Dispergier- oder Benetzungsmittel und/oder pharmakologische verträgliche Verdünnungsmittel, z. B. Propylen- oder Butylenglykol, und/oder Lösungsvermittler, z. B. Tweene, Cremophore oder Polyvinylpyrrolidon, enthalten.

Besonders vorteilhaft ist es, Lösungen der Komplexverbindungen in einem wasserfreien organischen Lösungsmittel mit Lösungen von hydrophilen Polymeren, wie beispielsweise Polyvinylpyrrolidonen (PVP) oder Polyoxyethylensorbitanfettsäureestern (Tween®) zu vermischen und die nach Abziehen des bzw. der Lösungsmittel zurückbleibenden Rückstände als wässerige Lösungen zu verabreichen. Als organische Lösungsmittel kommen beispielsweise Chloroform oder Methylenchlorid in Frage, die vor der Verwendung auf übliche Weise wasserfrei gemacht werden. Es hat sich als zweckmäßig erwiesen, die hydrophilen Polymeren in einem 5- bis 50-, vorzugsweise 10- bis 35-fachen gewichtsmäßigen Überschuß gegenüber der Komplexverbindung anzuwenden. Es ist auch möglich, die Polymeren als solche in Lösungen der Komplexverbindungen einzutragen. Den nach Abziehen des bzw. der Lösungsmittel verbleibenden Rückstand befreit man zweckmäßigerweise im Hochvakuum möglichst weitgehend von Lösungsmittelresten. Man erhält je nach Art und Menge des verwendeten hydrophilen Polymeren feste kristallartige oder glasartige oder auch flüssige oder klebrige Rückstände. Letztere lassen sich in der Regel durch Abkühlung in feste, meist wachsartige Produkte überführen. Im Sinne der vorliegenden Erfindung sollen diese Rückstände als Kopräzipitate bezeichnet werden. Die Herstellung der wäßrigen Lösungen erfolgt durch Behandeln der Kopräzipitate mit Wasser. PVP-Kopräzipitate gehen in der Regel bereits bei Raumtemperatur in Lösung. Kopräzipitate mit Polyoxyethylensorbitanfettsäureestern sind vorteilhaft in Lösung zu bringen, wenn man das Kopräzipitat und das Wasser vor dem Zusammengeben auf 25 bis 60 °C, vorzugsweise 30 bis 40 °C, erwärmt. Kopräzipitate, erhalten aus den Komplexverbindungen der weiter vorn angegebenen allgemeinen Formel I, worin M, $R^1$, $R^2$, $R^3$, X, m und n die dort angegebenen Bedeutungen haben, und hydrophilen Polymeren, Verfahren zur Herstellung dieser Kopräzipitate, sowie wäßrige Lösungen enthaltend diese Kopräzipitate, sind weitere bevorzugte Gegenstände der vorliegenden Erfindung.

Der Wirkstoff kann gegebenenfalls mit einem oder mehreren der angegebenen Träger- oder Zusatzstoffe auch in mikroverkapselter Form formuliert werden.

Die Herstellung der Komplexverbindungen erfolgt nach an sich bekannten Methoden durch Umsetzung von Metalltetrahalogenid mit dem entsprechenden Diketon unter sorgfältigem Feuchtigkeitsausschluß in inerten Lösungsmitteln, wie beispielsweise Benzol, n-Hexan, Diethylether, Methylenchlorid oder Chloroform. [Zirkon- und Hafniumkomplexe : M. Cox, J. Lewis u. R. S. Nyholm, J. Chem. Soc. 1964, 6113 ; Zinnkomplexe : W. Dilthey, J. Prakt. Chemie [2] *111*, 147 (1925) ; W. Dilthey, Ber. Deutsch. Chem. Ges. *36*, 923 (1903) ; G.T. Morgan, H.D.K. Drew, J. Chem. Soc. *1924*, 373 ; W.H. Nelson, Inorgan. Chem. *6*, 1509 (1967) ; R.W. Jones, Jr. u. R.C. Fay, Inorg. Chem. 12, 2599 (1973) ; Titankomplexe : N. Serpone u. R.C. Fay, Inorgan. Chem. *6*, 1835 (1967)].

Es ist zweckmäßig, die Reaktion in trockener Schutzgasatmosphäre, wie beispielsweise Stickstoff, durchzuführen. Feste Metalltetrahalogenide legt man als Aufschlämmung im gleichen Lösungsmittel vor, in dem das Diketon gelöst und zugetropft wird. Bei der Umsetzung von flüssigen Metalltetrahalogeniden, wie z. B. Titantetrachlorid, legt man zweckmäßigerweise das Diketon gelöst in einem inerten Lösungsmittel vor und tropft das Metalltetrachlorid zu. Es hat sich als günstig erwiesen, vom Diketon die theoretisch erforderliche Menge bis zu einem 20 %igen Überschuß vorzugsweise etwa 10 %igen Überschuß zuzugeben. Je nach Heftigkeit der Umsetzung wird bei Zimmertemperatur, unter Kühlung oder Erhitzen, beispielsweise am Rückfluß gearbeitet. Zur Vervollständigung der Umsetzung kann es notwendig sein, das Reaktionsgemisch ein bis drei Tage am Rückfluß zu erhitzen. Es hat sich als vorteilhaft erwiesen, den sich bei der Reaktion bildenden Halogenwasserstoff durch Durchleiten von trockenem Stickstoff durch das Reaktionsgemisch zu vertreiben. Nach Beendigung der Reaktion werden gegebenenfalls nicht umgesetzte feste Reste des Tetrahalogenids oder eventuell ungelöste Hydrolyseprodukte unter Schutzgasatmosphäre abgenutscht, bevor die Reaktionslösung zum Ausfällen des Produkts entweder eingeengt wird oder abgekühlt wird.

Die 1,3-Diketone sind bekannt, oder können nach an sich bekannten Methoden hergestellt werden. Beispielsweise können sie durch Esterkondensation des entsprechenden Arylmethylketons mit Essigsäureethylester bzw. des entsprechenden Arylessigsäureethylesters mit Natriumamid als Kondensationsmittel (J.T. Adams, C.R. Hauser, J. Amer. Chem. Soc. *66*, 1220 [1944]) erhalten werden. Weiter ist es möglich, durch Zugabe des entsprechenden Arylmethylketons, gelöst in Essigsäureethylester, zu einer Suspension von Natrium in Benzol oder Toluol zu den 1,3-Diketonen zu gelangen (D. W. Brown, S.F. Dyke, M. Sainsbury, G. Hardy, J. Chem. Soc. (c) *1971*, 3219). Eine weitere Möglichkeit besteht in der Umsetzung von Arylmethylketonen mit Acetanhydrid in Gegenwart von Bortrifluorid [H.G. Walker, C.R. Hauser, J. Amer. Chem. Soc. *68*, 2742 (1946)]. 2-Chlor-1,3-diketone werden durch Chlorierung des entsprechenden 1,3-Diketons mit Sulfurylchlorid in Ether [Macbeth, J. Chem. Soc. *123*, I, 1129 (1923)] hergestellt. 1-Benzyl-1,3-diketone werden durch Kondensation des entsprechenden Phenylessigsäureethylesters mit dem entsprechenden Methylketon in Gegenwart von Natriumamid hergestellt (A. Becker, Helv. Chin. Acta *149*, 1114 (1949)].

Die folgenden Beispiele erläutern die Erfindung näher ohne sie einzuschränken. Unter « Ether » wird « Diethylether » verstanden.

## Herstellungsbeispiele

### 1. Dibromobis(benzoylacetonato) zirkon(IV)

Zu einer Aufschlämmung von 10 g Zirkontetrabromid in 200 ml trockenem Ether werden 24 g Benzoylaceton, gelöst in 100 ml trockenem Ether, unter einem trockenen Stickstoffstrom zugetropft, wobei das Reaktionsgemisch am Rückfluß siedet. Das Reaktionsgemisch wird 24 Stunden am Rückfluß erhitzt. Der sich bildende Niederschlag wird zweimal mit je 100 ml Ether gewaschen und in ungefähr 250 ml Chloroform gelöst. Diese Lösung wird bis zur beginnenden Trübung eingeengt. Beim Abkühlen auf Raumtemperatur fällt ein farbloser Kristallbrei aus. Nach dreimaligem Umkristallisieren erhält man 1 g (8,5 % der Theorie) der Titelverbindung vom Schmelzpunkt 220-221 °C (rote Schmelze).

### 2. Bromotris(benzoylacetonato) zirkon(IV)

Zu einer Aufschlämmung von 11 g Zirkontetrabromid in 200 ml trockenem Benzol werden unter einem Stickstoffstrom und Sieden am Rückfluß 13 g Benzoylaceton, gelöst in 100 ml trockenem Benzol, zugetropft. Die Reaktionsmischung wird bis zur Beendigung der Bromwasserstoffentwicklung am Rückfluß gekocht. Aus der resultierenden rotbraunen Lösung fallen beim Abkühlen hellbraune Kristalle aus. Diese werden solange unter Rückfluß mit Ether behandelt, bis sich der Ether nicht mehr gelblich verfärbt. Nach Trocknen der Kristalle am Ölpumpenvakuum erhält man 10 g (57 % der Theorie) der Titelverbindung vom Schmelzpunkt 156 °C.

### 3. Dichlorobis(benzoylacetonato) hafnium(IV)

Zu einer Aufschlämmung von 5 g Hafniumtetrachlorid in 200 ml trockenem Ether werden unter einem Stickstoffstrom und unter Sieden am Rückfluß 15,2 g Benzoylaceton, gelöst in 100 ml trockenem Ether, zugetropft. Nach 24-stündigem Kochen am Rückfluß wird der gebildete Niederschlag abfiltriert und zweimal mit je 100 ml Ether gewaschen und in ungefähr 300 ml Chloroform gelöst. Nach dem Einengen bis zum Auftreten einer Trübung wird filtriert und die Lösung bei – 5 °C stehengelassen. Der farblose Niederschlag wird aus Chloroform umkristallisiert. Man erhält 3 g (33,7 % der Theorie) vom Schmelzpunkt 231-232 °C (rote Schmelze).

4. Dibromobis(benzoylacetonato) hafnium(IV)

Zu einer Aufschlämmung von 2 g Hafniumtetrabromid in 100 ml trockenem Ether werden unter einem Stickstoffstrom und Kochen am Rückfluß 2 g Benzoylaceton, gelöst in 100 ml trockenem Ether, zugetropft. Nach beendigter Bromwasserstoffentwicklung wird der rosafarbene Niederschlag abfiltriert und fünfmal mit Ether unter Rückfluß jeweils drei Stunden extrahiert. Man erhält 1 g (25 % der Theorie) der Titelverbindung.

5. Dibromobis(benzoylacetonato) titan(IV)

7,0 g (0,019 Mol) Titantetrabromid, gelöst in 40 ml trockenem Benzol, werden zu 8,0 g (0,050 Mol) Benzoylaceton, gelöst in 100 ml trockenem Benzol, gegeben. Das Reaktionsgemisch wird 30 Minuten unter Rückfluß erhitzt, wobei ein trockener Stickstoffstrom durch die Lösung geleitet wird. Nach Abdestillieren von etwa 40 ml Lösungsmittel wird das bräunlich-rote Produkt gesammelt und zweimal aus einem Benzol/Hexangemisch umkristallisiert und im Vakuum bei 80 °C getrocknet. Schmelzpunkt 210-222 °C.

6. Difluorobis(benzoylacetonato) titan(IV)

17,4 g (0,108 Mol) Benzoylaceton, gelöst in 85 ml Methylenchlorid, werden unter Rühren langsam zu einer Suspension von 5,40 g (0,043 6 Mol) Titantetrafluorid in etwa 350 ml Methylenchlorid zugegeben. Durch das Reaktionsgemisch wird unter Rühren ein langsamer Strom von trockenem Stickstoff geleitet bis die Entwicklung von Fluorwasserstoff beendet ist, was etwa 20 Stunden dauert. Die gelb-orange Lösung wird filtriert, dann unter vermindertem Druck auf ungefähr das halbe Volumen eingeengt. Beim Abkühlen kristallisiert ein gelbes Produkt aus, das abfiltriert und im Vakuum getrocknet wird (Ausbeute 11,0 g was 62 % der theoretisch berechneten Menge entspricht). Schmelzpunkt : 196-198,5 °C.

7. Dichlorobis(benzoylacetonato) titan(IV)

2 ml (0,018 Mol) frisch destilliertes Titantetrachlorid werden zu einer Lösung von 8 g (0,05 Mol) Benzoylaceton in 60 ml Benzol (getrocknet über Natriumdraht und durch azeotrope Destillation mit Ethanol) gegeben, wobei sich das Reaktionsgemisch erhitzt und ein dichter Rauch von Chlorwasserstoff entweicht. Das Reaktionsgemisch färbt sich dabei langsam tiefrot. Nachdem die Reaktion nachgelassen hat, wird die Lösung solange am Rückfluß gekocht, bis das gesamte Chlorwasserstoffgas entfernt ist (ungefähr 2,5 Stunden). Beim Abkühlen fallen orangefarbene Kristalle aus. Nach Abgießen der überstehenden Lösungen werden die Kristalle mit Benzol gewaschen und unter vermindertem Druck bei Raumtemperatur getrocknet.

Ausbeute : 11,68 g gelborange Kristalle. Schmelzpunkt : 209-210 °C (Zersetzung).

Die Identifizierung erfolgt spektroskopisch, beispielsweise durch NMR.

Weitere Herstellungsbeispiele samt Analysenwerten und Schmelzpunkten sind der folgenden Aufstellung zu entnehmen :

(Siehe Tabelle Seite 8 f.)

| $[R^1C(O)CR^3C(O)R^2]_2TiX_2$ | | | | Analyse: berechnet<br>gefunden | | | Schmelzpunkt<br>[°C]<br>(Zersetzung) |
|---|---|---|---|---|---|---|---|
| $R^1$ | $R^2$ | $R^3$ | X | C | H | X | |
| 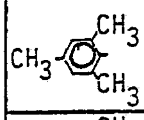 | $CH_3$ | H | F | 63,4<br>63,02 | 6,09<br>6,15 | 7,72<br>8,02 | 210-216 |
| 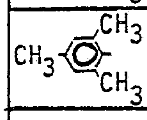 | $CH_3$ | H | Cl | 59,43<br>59,46 | 5,71<br>5,75 | 13,52<br>13,25 | 165-175 |
| 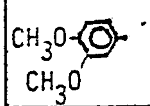 | $CH_3$ | H | F | 54,55<br>54,01 | 4,92<br>4,91 | 7,7<br>7,2 | 226-229 |
| 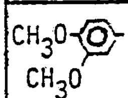 | $CH_3$ | H | Cl | 51,35<br>51,8 | 4,64<br>4,75 | | 215-220 |
| 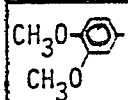 | $CH_3$ | H | Br | 44,32<br>44,47 | 4,0<br>4,04 | | 200 |
| 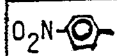 | $CH_3$ | H | F | 48,19<br>47,6 | 3,21<br>3,37 | | 200-203 |
| 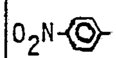 | $CH_3$ | H | Cl | 45,19<br>45,90 | 3,01<br>3,23 | | 190-202 |
| 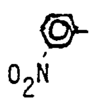 | $CH_3$ | H | F | 48,19<br>48,67 | 3,21<br>3,41 | | 209-221 |
| 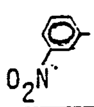 | $CH_3$ | H | Cl | 45,19<br>45,19 | 3,01<br>3,33 | | 212-217 |
| 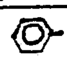 | $CH_3$ | Cl | Cl | 47,06<br>47,45 | 3,14<br>3,33 | | 160-162 |
| 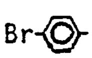 | $CH_3$ | H | F | 42,43<br>41,86 | 2,83<br>2,86 | | 189-193 |
| 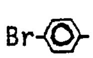 | $CH_3$ | H | Cl | 40,01<br>40,25 | 2,82<br>2,7 | | 230-238 |
| 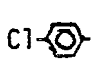 | $CH_3$ | H | Cl | 47,1<br>47,38 | 3,1<br>3,31 | | 216-220 |
| 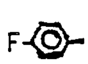 | $CH_3$ | H | F | 54.05<br>53,47 | 3,6<br>3,81 | | 220-225 |
| 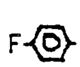 | $CH_3$ | H | Cl | 50,31<br>50,45 | 3,35<br>3,63 | | 210-220 |
| 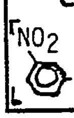$-C(O)CHC(O)CH_3]_2SnCl_2$ | | | | 39,92<br>39,42 | 2,66<br>2,67 | | > 250 |

8. Kopräzipitate von Dichlorobis(benzoylacetonato) titan(IV)

100 mg Dichlorobis(benzoylacetonato) titan(IV) werden in etwa 10 ml wasserfreiem Methylenchlorid gelöst. Zu der klaren Lösung gibt man eine Lösung von 1 g PVP 30 BT (Polyvinylpyrrolidon mit einem durchschnittlichen Molekulargewicht von 38 000 bis 40 000 und einem Viskositäts-K-Wert von 30) oder 2 g Tween® 60 (Sorbitanmonostearat von Polyoxyethylen) in etwa 30 ml wasserfreiem Methylenchlorid. Die resultierende Lösung wird einige Minuten gerührt. Es ist alternativ auch möglich, die Komplexverbindung in entsprechend mehr Lösungsmittel zu lösen und das Polymere als solches in die Lösung einzutragen. Das Lösungsmittel wird am Rotationsverdampfer abgezogen. Lösungsmittelreste werden durch Anlegen eines Hochvakuums entfernt.

Mit PVP 30 BT erhält man eine rötlichgelbe kristallartige Masse, die sich bis etwa 8 mg pro ml in Wasser löst.

Mit Tween® 60 erhält man eine rote, ölige, honigähnliche Flüssigkeit, die beim Abkühlen zu einer klebrig-festen Masse erstarrt. um wäßrige Lösungen herzustellen, ist es zweckmäßig, das Kopräzipitat und das Wasser vor dem Zusammengeben auf 30 bis 40 °C zu erwärmen. Es lösen sich bis etwa 17 mg pro ml Wasser.

## Pharmakologie

A. Tumormodelle

1. Sarkom 180-Tumormodell

Ca. 6 Wochen alten 18 bis 20 g schweren weiblichen NMRI-Mäusen werden je ca. $10^6$ Sarkom 180-Tumorzellen in 0,2 ml physiologischer Kochsalzlösung intraperitoneal (i. p.) übertragen. Der Tumor wird auf auf dem selben Mäusestamm in Passage gehalten. Die Tumorzellen werden frisch getöteten Tieren unmittelbar vor der Transplantation entnommen. Beim Überimpfen werden die Tiere randomisiert. Pro Dosierung werden 6 Mäuse verwendet. Als Kontrollsubstanz werden Cisplatin bzw. Cyclophosphamid eingesetzt. Die Anzahl der Kontrollgruppen (unbehandelte) Tiere wird so gewählt, daß sie in etwa der Quadratwurzel aus der Gesamtzahl der Gruppen entspricht. Die Substanzen werden als Suspensionen in üblichen Lösungsvermittlern, wie beispielsweise Tween® (Polyoxyethylenderivate von Sorbitanestern), jeweils 24 Stunden nach der Transplantation intraperitoneal gespritzt.

2. L 1210 Leukämie-Tumormodell auf NMRI-Mäusen

Ca. 6 Wochen alten 18 bis 20 g schweren weiblichen NMRI-Mäusen werden je ca. $10^6$ frisch entnommene Tumorzellen in 0,2 ml physiologischer Natriumchloridlösung intraperitoneal überimpft. Der Tumor wird auf dem selben Mäusestamm in Passage gehalten. Die Anzahl der Tiere, die Größe der Kontrollgruppe und die Verabreichung der Substanzen entspricht der beim unter Punkt 1 beschriebenen Sarkom 180-Tumormodell.

3. Walker 256 Carcinosarkom-Tumormodell

Weiblichen ungefähr 150 g schweren SD-Ratten werden je ca. $10^6$ Tumorzellen in 0,5 ml physiologischer Kochsalzlösung intraperitoneal überimpft. Der Tumor wird auf Ratten desselben Stammes in Passage gehalten. Die Anzahl der Tiere und die Verabreichung der Substanzen entspricht der beim unter Punkt 1 beschriebenen Sarkom 180-Tumormodell. Pro Dosierung werden 6 Tiere verwendet.

B. Versuchsergebnisse

In der nachfolgenden Tabelle I sind Ergebnisse aus dem unter Punkt A 1 beschriebenen Sarkom 180-Tumormodell zusammengestellt. Die angegebene Dosis wurde einmalig zu Beginn des Versuchs wie angegeben appliziert. Der in Prozent angegebene Faktor T/C bedeutet die prozentuale Verlängerung der medianen Überlebenszeit der behandelten Tiere im Vergleich zur medianen Überlebenszeit der unbehandelten Kontrolltiere. Zum Teil ist in der Tabelle hinter dem Faktor T/C in Klammern die Heilungsrate, d. h. die Zahl der geheilten Tiere im Verhältnis zur Zahl der behandelten Tiere, angegeben.

Der Versuch wird abgebrochen sobald die mediane Überlebenszeit T/C der behandelten Tiere 300 % der medianen Überlebenszeit der unbehandelten Tiere erreicht hat. Zur Berechnung der medianen Überlebenszeit werden die bei Versuchsende noch lebenden Tiere als bei Versuchsende gestorben berücksichtigt. Die Vergleichsverbindung Cisplatin wirkt in einem Dosisbereich von etwa 8 bis 10 mg/kg therapeutisch und die Tiere werden zum großen Teil geheilt. Die mediane Überlebenszeit der mit Cisplatin behandelten Tiere wird bereits bei einer Dosis von etwa 20 mg/kg kleiner als die der unbehandelten Kontrolltiere.

Tabelle I

| $[R^1C(O)CR^3C(O)R^2]_kMX$ | | | | | | Dosis [mg/kg] | T/C [%] |
|---|---|---|---|---|---|---|---|
| $R^1$ | $R^2$ | $R^3$ | k | M | X | | |
| 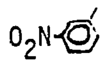 | $CH_3$ | H | 2 | Ti | $Cl_2$ | 160<br>229 | 115<br>188 |
| 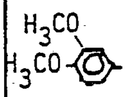 | $CH_3$ | H | 2 | Ti | $Cl_2$ | 168<br>241 | 191<br>300 |
| 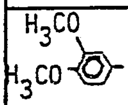 | $CH_3$ | H | 2 | Ti | $Br_2$ | 280 | 209 |
| 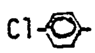 | $CH_3$ | H | 2 | Ti | $Cl_2$ | 102 | 173 |
| 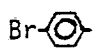 | $CH_3$ | H | 2 | Ti | $F_2$ | 244 | 300 |
| 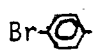 | $CH_3$ | H | 2 | Ti | $Cl_2$ | 180<br>258 | 300<br>300 |
| 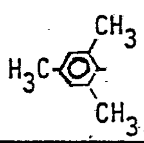 | $CH_3$ | H | 2 | Ti | $Cl_2$ | 158<br>226 | 300<br>300 |
| 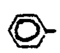 | $CH_3$ | H | 2 | Ti | $F_2$ | 4<br>15<br>38<br>52<br>75<br>113<br>162<br>238 | 97 (0/6)<br>115 (0/6)<br>209 (1/6)<br>300 (4/6)<br>154 (0/6)<br>300 (3/6)<br>300 (4/6)<br>300 (2/6) |
| 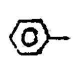 | $CH_3$ | H | 2 | Ti | $Br_2$- | 5<br>20<br>50<br>70<br>100<br>150<br>215<br>315 | 103 (0/6)<br>115 (0/6)<br>288 (1/6)<br>300 (3/6)<br>300 (3/6)<br>300 (4/6)<br>273 (1/6)<br>300 (5/6) |
| 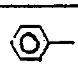 | $CH_3$ | H | 2 | Ti | $Cl_2$ | 4<br>16<br>41<br>57<br>82<br>123<br>177<br>259 | 118 (0/6)<br>115 (0/6)<br>291 (2/6)<br>179 (0/6)<br>300 (4/6)<br>212 (1/6)<br>251 (1/6)<br>300 (5/6) |

Tabelle I (Fortsetzung)

| $[R^1C(O)CR^3C(O)R^2]_kMX$ | | | | | | Dosis [mg/kg] | T/C [%] |
|---|---|---|---|---|---|---|---|
| 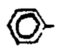 | 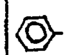 | H | 2 | Ti | $Br_2$ | 63<br>92<br>111 | 203<br>300<br>280 |
| 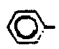 | 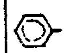 | H | 2 | Ti | $Cl_2$ | 37<br>54<br>79 | 293<br>273<br>266 |
| 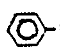$-CH_2-$ | 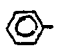 | H | 2 | Ti | $Cl_2$ | 60<br>119 | 178<br>300 |
| 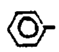 | $CH_3$ | H | 2 | Zr | $Cl_2$ | 31<br>46<br>68<br>97<br>145<br>208 | 234<br>300<br>300<br>300<br>193<br>144 |
| 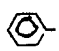 | $CH_3$ | H | 3 | Zr | Cl | 85<br>195<br>439 | 137<br>300<br>222 |
| 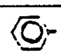 | $CH_3$ | H | 2 | Zr | $Br_2$ | 54<br>80<br>126<br>172<br>247 | 300<br>300<br>191<br>172<br>184 |
| 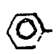 | $CH_3$ | H | 3 | Zr | Br | 92<br>209<br>471 | 300<br>297<br>187 |
| 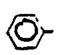 | $CH_3$ | H | 2 | Hf | $Cl_2$ | 114<br>172<br>246 | 130<br>267<br>300 |
| 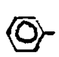 | $CH_3$ | H | 3 | Hf | Cl | 98<br>223<br>502 | 220<br>300<br>287 |
| 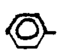 | $CH_3$ | H | 2 | Sn | $Cl_2$ | 52,6 | 230 |
| 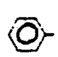 | $CH_3$ | H | 2 | Sn | $Br_2$ | 84 | 141 |
| Cisplatin | | | | | | 1<br>2<br>3<br>5<br>8<br>13<br>22 | 112 (0/6)<br>133 (0/6)<br>300 (2/6)<br>300 (5/6)<br>300 (6/6)<br>300 (6/6)<br>42 (0/6) |

In der nachfolgenden Abb. 1 sind die Ergebnisse von Vergleichsversuchen am L 1210 Leukämie-Tumormodell der Verbindungen Difluorobis-(benzoylacetonato) titan(IV) [Kurve (a)], Dichlorobis(benzoylacetonato)-titan(IV) [Kurve (b)] und Dibromobis(benzoylacetonato) titan(IV) [Kurve (c)] wiedergegeben. Auf der Ordinate ist die mediane Überlebenszeit in Tagen und auf der Abszisse die einmalig bei Versuchsbeginn verabreichte Dosis aufgetragen. Die mediane Überlebenszeit der unbehandelten Kontrolltiere (18 Tage) ist als waagerechte Linie eingetragen. Zusätzlich sind bei den einzelnen Dosierungen in Klammern die Heilungsrate (Zahl der geheilten Tiere pro Gruppe von 6 Tieren angegeben). Die Versuche wurden nach 60 Tagen abgebrochen. Als Vergleichssubstanz wurde Cisplatin in einer Dosis von 8 mg/kg verabreicht.

Abb. 1

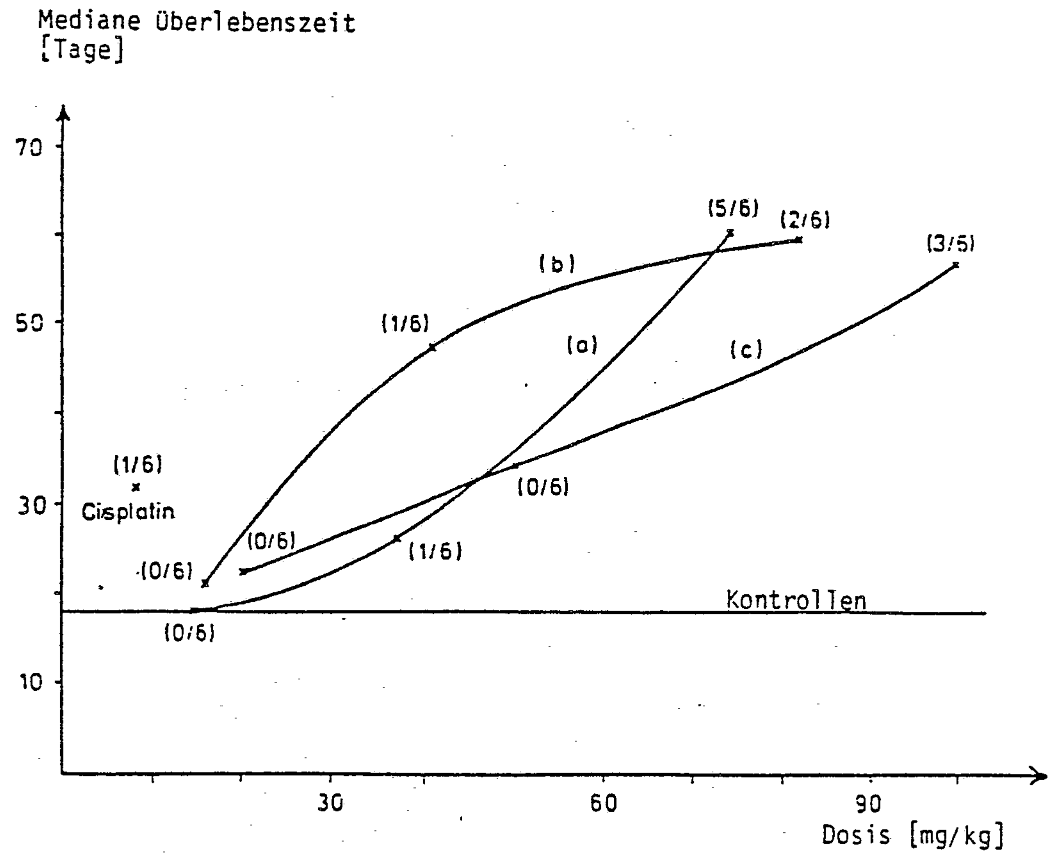


In der nachfolgenden Abb. 2 sind die Ergebnisse von Vergleichsversuchen am Walker 256 Carcinosarkom-Tumormodell der Verbindungen Difluorobis-(benzoylacetonato) titan (IV) [Kurve (a)], Dichlorobis(benzoylacetonato) titan (IV) [Kurve (b)] und Dibromobis(benzoylacetonato) titan (IV) [Kurve (c)] wiedergegeben. Auf der Ordinate ist die mediane Überlebenszeit in Tagen und auf der Abszisse die einmalig bei Versuchsbeginn verabreichte Dosis aufgetragen. Die mediane Überlebenszeit der unbehandelten Kontrolltiere (4,5 Tage) ist als waagerechte Linie eingetragen.

(Siehe Abbildung 2 Seite 13 f.)

Abb. 2

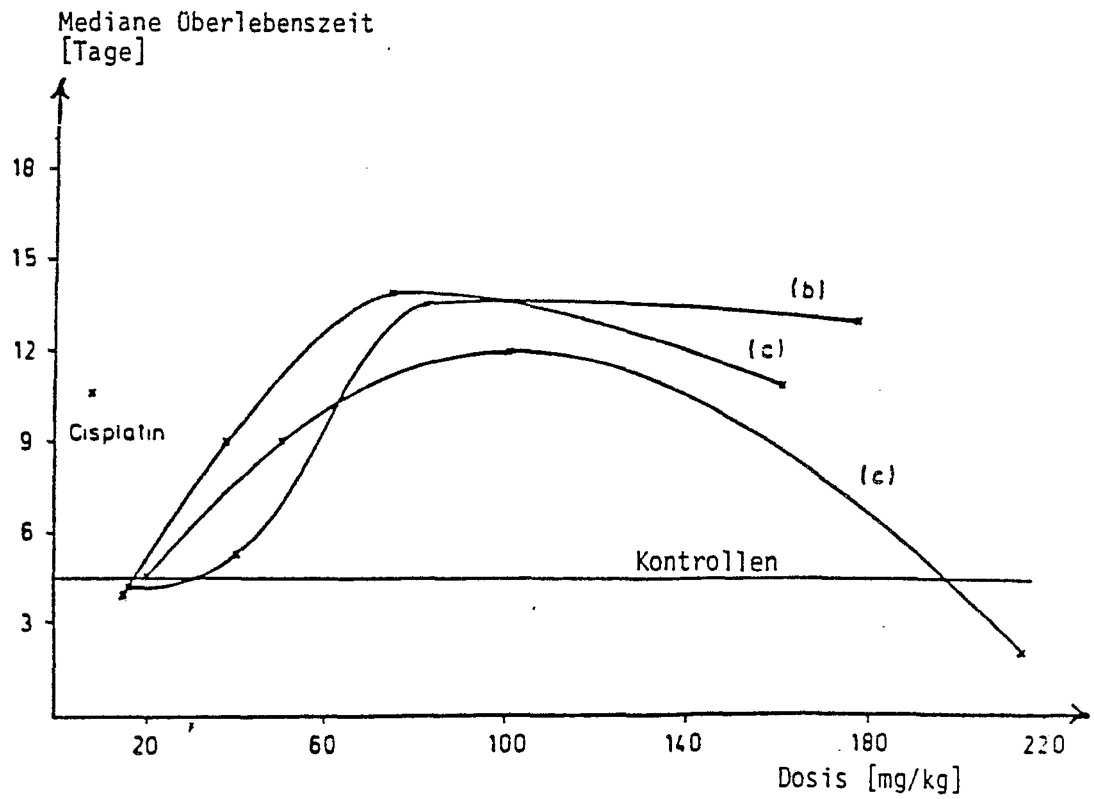


Aus diesen Untersuchungen ergibt sich, daß die untersuchten Komplexverbindungen bedeutend wirksamer als Cisplatin sind. Gleichzeitig ist ihre Toxizität im Vergleich zu Cisplatin ($LD_{50}$ bei Mäusen und Ratten 12 mg/kg) sehr viel geringer. Die von Cisplatin bekannte Nephrotoxizität konnte bei den untersuchten Komplexverbindungen nicht beobachtet werden.

In einem weiteren Versuch wurden zwölf weiblichen Mäusen am Tag 0 ca. $5 \times 10^6$ Sarkom 180-Zellen subkutan transplantiert. Sechs Tiere wurden ab der 2. Woche nach der Transplantation für 4 Wochen zweimal pro Woche mit 4 mg/kg Dichlorobis(benzoylacetonato) titan (IV) als Tween® 60-Copräzipitat (1 : 20) in wäßriger Lösung intravenös therapiert. Die behandelten Tiere waren nach Therapieende geheilt, während die unbehandelten Kontrolltiere etwa walnußgroße Sarkome aufwiesen, die meist bereits zentral nekrotisiert und an mehreren Stellen exulceriert waren.

In einem weiteren Versuch wurden weiblichen, SD-Ratten am Tag 0 ca. $5 \times 10^6$ Yoshida-Sarkom-Zellen subkutan transplantiert. In der zweiten Woche wurden zweimal 4 mg/kg Dichlorobis(benzoylacetonato) titan (IV) als Tween® 60-Copräzipitat (1 : 20) in wäßriger Lösung intravenös verabreicht. Der Tumor ging in Remission. Nach einer Pause von drei Wochen waren bei zwei von drei Tieren pflaumengroße Rezidive entstanden. Ein Tier war geheilt. Die Rezidive wurden vier Wochen lang mit der ursprünglichen Dosis zweimal pro Woche intravenös behandelt. Beide Tiere wurden geheilt. Die Kontrolltiere zeigten zu diesem Zeitpunkt ausgedehntes Tumorwachstum und starben kurze Zeit später.

**Patentansprüche** (für die Vertragsstaaten : BE, CH, DE, FR, GB, IT, LI, LU, NL, SE)

1. Komplexverbindungen der allgemeinen Formel I,

$$[R^1(CH_2)_mC(O)CR^3C(O)R^2]_{2+n}MX_{2-n} \quad (I),$$

worin

M Zinn, Titan, Zirkon oder Hafnium,

$R^1$ Wasserstoff oder einen Phenylrest, wobei der Phenylrest durch Fluor, Chlor, Brom, Nitro, $C_1$-$C_4$-Alkyl, $C_1$-$C_4$-Alkoxy oder Trifluormethyl einfach oder mehrfach substituiert sein kann,

$R^2$ Methyl oder $R^1$ mit Ausnahme von Wasserstoff,

$R^3$ Wasserstoff oder Chlor,

X Fluor, Chlor oder Brom, jedoch nicht Fluor wenn M die Bedeutung Zirkon oder Hafnium hat,

m die Zahl 1 oder 0 oder, falls $R^1$ die Bedeutung Wasserstoff hat, die Zahl 1 und

n die Zahl 0 oder, falls M die Bedeutung Zirkon oder Hafnium hat, die Zahl 1 oder 0, bedeuten, zur Anwendung bei der Behandlung von Krebskrankheiten.

2. Komplexverbindungen der allgemeinen Formel I'

$$[R^{1'}(CH_2)_{m'}C(O)CR^{3'}C(O)R^{2'}]_{2+n'}M'X'_{2-n'} \quad (I'),$$

worin

M' Zinn, Titan, Zirkon oder Hafnium,

$R^{1'}$ einen Phenylrest, der 4-Chlor, 4-Brom-, 3-Nitro-, 4-Methoxy-, 3,4-Dimethoxy-, 4-Methyl- oder 2,4,6-Trimethyl-substituiert sein kann,

$R^{2'}$ Methyl oder $R^{1'}$,

$R^{3'}$ Wasserstoff oder Chlor,

X' Fluor, Chlor oder Brom, jedoch nicht Fluor, wenn M' die Bedeutung Zirkon oder Hafnium hat,

m' die Zahl 1 oder 0 und

n' die Zahl 0 oder, falls M' die Bedeutung Zirkon oder Hafnium hat, die Zahl 1 oder 0, bedeuten, zur Anwendung bei der Behandlung von Krebskrankheiten.

3. Komplexverbindungen der allgemeinen Formel I"

$$[R^{1''}C(O)CR^{3''}C(O)R^{2''}]_{2+n''}M''X''_{2-n''} \quad (I''),$$

worin

M" Zinn, Titan, Zirkon oder Hafnium,

$R^{1''}$ einen Phenylrest,

$R^{2''}$ einen Methylrest,

$R^{3''}$ Wasserstoff oder Chlor,

X" Fluor, Chlor oder Brom, jedoch nicht Fluor wenn M" die Bedeutung Zirkon oder Hafnium hat, und

n" die Zahl 0 oder, falls M" die Bedeutung Zirkon oder Hafnium hat, die Zahl 1 oder 0, bedeuten, zur Anwendung bei der Behandlung von Krebskrankheiten.

4. Dichlorobis(benzoylacetonato) titan(IV), Dichlorobis(benzoylacetonato) zirkon(IV) und/oder Dichlorobis(benzoylacetonato) hafnium(IV) zur Anwendung bei der Behandlung von Krebskrankheiten.

5. Verbindungen der allgemeinen Formel $I^a$

$$[R^{1a}(CH_2)_{m^a}C(O)CR^{3a}C(O)R^{2a}]_{2+n^a}M^aX^a_{2-n^a} \quad (I^a),$$

worin

$M^a$ Zinn, Titan, Zirkon, Hafnium,

$R^{1a}$ einen durch Fluor, Chlor, Brom, Nitro, $C_1$-$C_4$-Alkyl, $C_1$-$C_4$-Alkoxy oder Trifluormethyl einfach oder mehrfach substituierten Phenylrest, und falls $M^a$ Zirkon und Hafnium darstellt zusätzlich einen Phenylrest,

$R^{2a}$ Methyl oder $R^{1a}$ einschließlich Phenyl für alle Bedeutungen von $M^a$,

$R^{3a}$ Wasserstoff oder Chlor,

$X^a$ Fluor, Chlor oder Brom,

jedoch nich Fluor wenn $M^a$ für Zirkon oder Hafnium und nicht Chlor, wenn $M^a$ für Titan oder Zinn und $R^{1a}$ für p-Fluor-, p-Chlor- oder p-Bromphenyl und $R^{2a}$ für Methyl oder wenn $M^a$ für Zirkon und $R^{1a}$ für Phenyl oder wenn $M^a$ für Hafnium und $R^{1a}$ für Phenyl und $R^{2a}$ für Methyl und $n^a$ für 1 oder wenn $M^a$ für Sn und $R^{1a}$ und $R^{2a}$ für Phenyl, und nicht Brom, wenn $M^a$ für Zirkon oder Hafnium und $R^{1a}$ und $R^{2a}$ für Phenyl, oder wenn $M^a$ für Zinn und $R^{1a}$ für Phenyl oder p-Fluorphenyl, und $R^{2a}$ für Methyl stehen,

$m^a$ die Zahl 1 oder 0, und

$n^a$ die Zahl 0 oder, falls $M^a$ die Bedeutung Zirkon oder Hafnium hat, die Zahl 1 oder 0, bedeuten.

6. Verbindungen der allgemeinen Formel $I^{a'}$

$$[R^{1a'}(CH_2)_{m^{a'}}C(O)CR^{3a'}C(O)R^{2a'}]_{2+n^{a'}}M^{a'}M^{a'}X^{a'}_{2-n^{a'}} \quad (I^{a'}),$$

worin

$M^{a'}$ Zirkon oder Hafnium,

$R^{1a'}$ Phenyl,

$R^{2a'}$ Methyl,

$R^{3a'}$ Wasserstoff oder Chlor,

$X^{a'}$ Chlor oder Brom, aber nicht Chlor, falls $M^{a'}$ für Zirkon oder falls $M^{a'}$ für Hafnium und $n^{a'}$ für die Zahl 1 stehen,

$m^{a'}$ die Zahl 1 oder 0 und

$n^{a'}$ die Zahl 1 oder 0 bedeuten.

7. Dichlorobis(benzoylacetonato) hafnium(IV).

8. Dibromobis(benzoylacetonato) zirkon(IV).

9. Verfahren zur Herstellung der Verbindungen nach Anspruch 5 oder 6 der allgemeinen Formeln $I^a$ bzw. $I^{a'}$, worin $R^{1a}$ bzw. $R^{1a'}$, $R^{2a}$ bzw. $R^{2a'}$, $R^{3a}$ bzw. $R^{3a'}$, $X^a$ bzw. $X^{a'}$, $m^a$ bzw. $m^{a'}$ und $n^a$ bzw. $n^{a'}$ die in Anspruch 5 bzw. 6 angegebenen Bedeutungen haben, das dadurch gekennzeichnet ist, daß man ein Metalltetrahalogenid $M^aX_4{}^a$ bzw. $M^{a'}X_4{}^{a'}$, wobei $M^a$ bzw. $M^{a'}$ und $X^a$ bzw. $X^{a'}$ die in Anspruch 5 bzw. 6 angegebenen Bedeutungen haben, unter Feuchtigkeitsausschluß in einem inerten Lösungsmittel mit einem Diketon $R^{1a}(CH_2)_{m^a}C(O)CHR^{3a}C(O)R^{2a}$ bzw. $R^{1a'}(CH_2)_{m^{a'}}C(O)CHR^{3a'}C(O)R^{2a'}$, wobei $R^{1a}$ bzw. $R^{1a'}$, $R^{2a}$ bzw. $R^{2a'}$, $R^{3a}$ bzw. $R^{3a'}$ und $m^a$ bzw. $m^{a'}$ die in Anspruch 5 bzw. 6 angegebenen Bedeutungen haben, im molaren Verhältnis von höchstens 1 : 2 umsetzt.

10. Verfahren nach Anspruch 9 zur Herstellung von Dichlorobis(benzoylacetonato) hafnium(IV), dadurch gekennzeichnet, daß man Hafniumtetrachlorid mit Benzoylaceton umsetzt.

11. Verfahren nach Anspruch 9 zur Herstellung von Dibromobis(benzoylacetonato)-zirkon(IV), dadurch gekennzeichnet, daß man Zirkontetrabromid mit Benzoylaceton umsetzt.

12. Arzneimittel enthaltend eine oder mehrere Komplexverbindungen nach einem oder mehreren der Ansprüche 5 bis 8.

13. Verbindungen nach einem oder mehreren der Ansprüche 5 bis 8 zur Anwendung bei der Behandlung von Krebskrankheiten.

14. Kopräzipitate erhalten aus den Komplexverbindungen der allgemeinen Formel I

$$[R^1(CH_2)_mC(O)CR^3C(O)R^2]_{2+n}MX_{2-n} \qquad (I),$$

worin

M Zinn, Titan, Zirkon oder Hafnium,

$R^1$ Wasserstoff oder einen Phenylrest, wobei der Phenylrest durch Fluor, Chlor, Brom, Nitro, $C_1$-$C_4$-Alkyl, $C_1$-$C_4$-Alkoxy oder Trifluormethyl einfach oder mehrfach substituiert sein kann,

$R^2$ Methyl oder $R^1$ mit Ausnahme von Wasserstoff,

$R^3$ Wasserstoff oder Chlor,

X Fluor, Chlor oder Brom, jedoch nicht Fluor wenn M die Bedeutung Zirkon oder Hafnium hat,

m die Zahl 1 oder 0 oder, falls $R^1$ die Bedeutung Wasserstoff hat, die Zahl 1 und

n die Zahl 0 oder, falls M die Bedeutung Zirkon oder Hafnium hat, die Zahl 1 oder 0, bedeuten und hydrophilen Polymeren.

15. Kopräzipitate nach Anspruch 14, dadurch gekennzeichnet, daß es sich bei den hydrophilen Polymeren um Polyvinylpyrrolidon handelt.

16. Kopräzipitate nach Anspruch 14, dadurch gekennzeichnet, daß es sich bei den hydrophilen Polymeren um Polyoxyethylensorbitanmonofettsäureester handelt.

17. Kopräzipitate nach Anspruch 16, dadurch gekennzeichnet, daß der Polyoxyethylensorbitanmonofettsäureester Polyoxyethylen (20) sorbitanmonostearat ist.

18. Kopräzipitat nach Anspruch 16, dadurch gekennzeichnet, daß der Polyoxyethylensorbitanmonofettsäureester Polyoxyethylen (20) sorbitanmonooleat ist.

19. Kopräzipitat aus Dichlorobis(benzoylacetonato) titan(IV) und Polyoxyethylen (20) sorbitanmonostearat.

20. Kopräzipitat aus Dichlorobis(benzoylacetonato) titan(IV) und Polyoxyethylen (20) sorbitanmonooleat.

21. Verfahren zur Herstellung von Kopräzipitaten nach einem der Ansprüche 14 bis 20, dadurch gekennzeichnet, daß man eine Lösung einer Komplexverbindung der allgemeinen Formel I

$$[R^1(CH_2)_mC(O)CR^3C(O)R^2]_{2+n}MX_{2-n} \qquad (I),$$

worin

M Zinn, Titan, Zirkon oder Hafnium,

$R^1$ Wasserstoff oder einen Phenylrest, wobei der Phenylrest durch Fluor, Chlor, Brom, Nitro, $C_1$-$C_4$-Alkyl, $C_1$-$C_4$-Alkoxy oder Trifluormethyl einfach oder mehrfach substituiert sein kann,

$R^2$ Methyl oder $R^1$ mit Ausnahme von Wasserstoff,

$R^3$ Wasserstoff oder Chlor,

X Fluor, Chlor oder Brom, jedoch nicht Fluor wenn M die Bedeutung Zirkon oder Hafnium hat,

m die Zahl 1 oder 0 oder, falls $R^1$ die Beteutung Wasserstoff hat, die Zahl 1 und

n die Zahl 0 oder, falls M die Bedeutung Zirkon oder Hafnium hat, die Zahl 1 oder 0, bedeuten, und eines hydrophilen Polymeren in einem inerten organischen Lösungsmittel bis zur Trockene einengt.

22. Sterile wässerige Lösungen enthaltend Kopräzipitate nach einem der Ansprüche 14 bis 20.

**Patentansprüche** (für den Vertragsstaat AT)

1. Verfahren zur Herstellung von antineoplastisch wirksamen Mitteln, dadurch gekennzeichnet, daß man Komplexverbindungen der allgemeinen Formel I,

$$[R^1(CH_2)_mC(O)CR^3C(O)R^2]_{2+n}MX_{2-n} \quad (I),$$

worin

M Zinn, Titan, Zirkon oder Hafnium,

$R^1$ Wasserstoff oder einen Phenylrest, wobei der Phenylrest durch Fluor, Chlor, Brom, Nitro, $C_1$-$C_4$-Alkyl, $C_1$-$C_4$-Alkoxy oder Trifluormethyl einfach oder mehrfach substitutiert sein kann,

$R^2$ Methyl oder $R^1$ mit Ausnahme von Wasserstoff,

$R^3$ Wasserstoff oder Chlor,

X Fluor, Chlor oder Brom, jedoch nicht Fluor, wenn M die Bedeutung Zirkon oder Hafnium hat,

m die Zahl 1 oder 0 oder, falls $R^1$ die Bedeutung Wasserstoff hat, die Zahl 1, und

n die Zahl 0 oder, falls M die Bedeutung Zirkon oder Hafnium hat, die Zahl 1 oder 0, bedeuten, herstellt, indem man ein Metalltetrahalogenid $MX_4$, wobei M und X die in Formel I angegebenen Bedeutungen haben, unter Feuchtigkeitsausschluß in einem inerten Lösungsmittel mit einem Diketon $R^1(CH_2)_mC(O)CHR^3C(O)R^2$, wobei $R^1$, $R^2$, $R^3$ und m die in Formel I angegebenen Bedeutungen haben, im molaren Verhältnis von höchstens 1 : 2 umsetzt, und daß eine oder mehrere der so erhaltenen Komplexverbindungen der Formel I durch Mischen mit einem geeigneten pharmazeutisch annehmbaren Trägerstoff in eine zur Verabreichung als antineoplastisch wirksames Mittel geeignete Form gebracht werden.

2. Verfahren zur Herstellung von antineoplastisch wirksamen Mitteln, dadurch gekennzeichnet, daß man Komplexverbindungen der allgemeinen Formel I'

$$[R^{1'}(CH_2)_{m'}C(O)CR^{3'}C(O)R^{2'}]_{2+n'}M'X'_{2-n'} \quad (I'),$$

worin

M' Zinn, Titan, Zirkon oder Hafnium,

$R^{1'}$ einen Phenylrest, der 4-Chlor, 4-Brom, 3-Nitro, 4-Methoxy-, 3,4-Dimethoxy-, 4-Methyl- oder 2,4,6-Trimethyl-substituiert sein kann,

$R^{2'}$ Methyl oder $R^{1'}$,

$R^{3'}$ Wasserstoff oder Chlor,

X' Fluor, Chlor oder Brom, jedoch nicht Fluor, wenn M' die Bedeutung Zirkon oder Hafnium hat,

m' die Zahl 1 oder 0 und

n' die Zahl 0 oder, falls M' die Beteutung Zirkon oder Hafnium hat, die Zahl 1 oder 0, bedeuten, herstellt, indem man ein Metalltetrahalogenid $M'X'_4$, wobei M' und X' die in Formel I' angegebenen Bedeutungen haben, unter Feuchtigkeitsausschluß in einem inerten Lösungsmittel mit einem Diketon $R^{1'}(CH_2)_{m'}C(O)CHR^{3'}C(O)R^{2'}$, wobei $R^{1'}$, $R^{2'}$, $R^{3'}$ und m' die in Formel I' angegebenen Bedeutungen haben, im molaren Verhältnis von höchstens 1 : 2 umsetzt, und daß eine oder mehrere der so erhaltenen Komplexverbindungen der Formel I' durch Mischen mit einem geeigneten pharmazeutisch annehmbaren Trägerstoff in eine zur Verabreichung als antineoplastisch wirksames Mittel geeignete Form gebracht werden.

3. Verfahren zur Herstellung von antineoplastisch wirksamen Mitteln, dadurch gekennzeichnet, daß man Komplexverbindungen der allgemeinen Formel I"

$$[R^{1''}C(O)CR^{3''}C(O)R^{2''}]_{2+n''}M''X''_{2-n''} \quad (I''),$$

worin

M" Zinn, Titan, Zirkon oder Hafnium,

$R^{1''}$ einen Phenylrest,

$R^{2''}$ einen Methylrest

$R^{3''}$ Wasserstoff oder Chlor,

X" Fluor, Chlor oder Brom, jedoch nicht Fluor, wenn M" die Bedeutung Zirkon oder Hafnium hat,

n" die Zahl 0 oder, falls M" die Bedeutung Zirkon oder Hafnium hat, die Zahl 1 oder 0, bedeuten, herstellt, indem man ein Metalltetrahalogenid $M''X''_4$, wobei M" und X" die in Formel I" angegebenen Bedeutungen haben, unter Feuchtigkeitsausschluß in einem inerten Lösungsmittel mit einem Diketon $R^{1''}C(O)CHR^{3''}C(O)R^{2''}$, wobei $R^{1''}$, $R^{2''}$ und $R^{3''}$ die in Formel I" angegebenen Bedeutungen haben, im molaren Verhältnis von höchstens 1 : 2 umsetzt, und daß eine oder mehrere der so erhaltenen Komplexverbindungen der Formel I" durch Mischen mit einem geeigneten pharmazeutisch annehmbaren Trägerstoff in eine zur Verabreichung als antineoplastisch wirksames Mittel geeignete Form gebracht werden.

4. Verfahren zur Herstellung von antineoplastisch wirksamen Mitteln, dadurch gekennzeichnet, daß man die Komplexverbindung Dichlorobis(benzoylacetonato) titan(IV) herstellt, indem man Titan(IV) tetrachlorid unter Feuchtigkeitsausschluß in einem inerten Lösungsmittel mit Benzoylaceton im molaren Verhältnis von höchstens 1 : 2 umsetzt, und daß Dichlorobis(benzoylacetonato) titan(IV) durch Mischen mit einem geeigneten pharmazeutisch annehmbaren Trägerstoff in eine zur Verabreichung als antineoplastisch wirksames Mittel geeignete Form gebracht wird.

5. Verfahren zur Herstellung von Verbindungen der allgemeinen Formel $I^a$

$$[R^{1a}(CH_2)_{ma}C(O)CR^{3a}C(O)R^{3a}]_{2+na}M^aX^a{}_{2-na} \quad (I^a).$$

worin

$M^a$ Zinn, Titan, Zirkon oder Hafnium,

$R^{1a}$ einen durch Fluor, Chlor, Brom, Nitro, $C_1$-$C_4$-Alkyl, $C_1$-$C_4$-Alkoxy oder Trifluormethyl einfach oder mehrfach substitutierten Phenylrest, und falls $M^a$ Zirkon oder Hafnium darstellt zusätzlich einen Phenylrest,

$R^{2a}$ Methyl oder $R^{1a}$ einschließlich Phenyl für alle Bedeutungen von $M^a$,

$R^{3a}$ Wasserstoff oder Chlor,

$X^a$ Fluor, Chlor oder Brom,

jedoch nicht Fluor, wenn $M^a$ für Zirkon oder Hafnium, und nicht Chlor, wenn $M^a$ für Titan oder Zinn und $R^{1a}$ für p-Fluor-, p-Chlor oder p-Bromphenyl und $R^{2a}$ für Methyl oder wenn $M^a$ für Zirkon und $R^{1a}$ für Phenyl oder wenn $M^a$ für Hafnium und $R^{1a}$ für Phenyl und $R^{2a}$ für Methyl und $n^a$ für 1 oder wenn $M^a$ für Sn und $R^{1a}$ und $R^{2a}$ für Phenyl und nicht Brom, wenn $M^a$ für Zirkon oder Hafnium und $R^{1a}$ und $R^{2a}$ für Phenyl, oder wenn $M^a$ für Zinn und $R^{1a}$ für Phenyl oder p-Fluorphenyl, und $R^{2a}$ für Methyl stehen,

$m^a$ die Zahl 1 oder 0, und

$n^a$ die Zahl 0 oder, falls $M^a$ die Bedeutung Zirkon oder Hafnium hat, die Zahl 1 oder 0, bedeuten, das dadurch gekennzeichnet ist, daß man ein Metallhalogenid $M^aX^a{}_4$, wobei $M^a$ und $X^a$ die oben angegebenen Bedeutungen haben, unter Feuchtigkeitsausschluß in einem inerten Lösungsmittel mit einem Diketon $R^{1a}(CH_2)_{ma}C(O)CHR^{3a}C(O)R^{2a}$, wobei $R^{1a}$, $R^{2a}$, $R^{3a}$ und $m^a$ die oben angegebenen Bedeutungen haben, im molaren Verhältnis von höchstens 1 : 2 umsetzt.

6. Verfahren zur Herstellung von Verbindungen der allgemeinen Formel $I^{a'}$

$$[R^{1a'}(CH_2)_{ma'}C(O)CR^{3a'}C(O)R^{2a'}]_{2+na'}M^{a'}X^{a'}{}_{2-na'} \quad (I^{a'}),$$

worin

$M^{a'}$ Zirkon oder Hafnium,

$R^{1a'}$ Phenyl

$R^{2a'}$ Methyl,

$R^{3a'}$ Wasserstoff oder Chlor,

$X^{a'}$ Chlor oder Brom, aber nicht Chlor, wenn $M^{a'}$ für Zirkon oder $M^{a'}$ für Hafnium und und $n^{a'}$ für die Zahl 1 stehen,

$m^{a'}$ die Zahl 1 oder 0, und

$n^{a'}$ die Zahl 0 oder, falls $M^{a'}$ die Bedeutung Zirkon oder Hafnium hat, die Zahl 1 oder O, bedeuten, das dadurch gekennzeichnet ist, daß man ein Metallhalogenid $M^{a'}X^{a'}{}_4$, wobei $M^{a'}$ und $X^{a'}$ die oben angegebenen Bedeutungen haben, unter Feuchtigkeitsausschluß in einem inerten Lösungsmittel mit einem Diketon $R^{1a'}(CH_2)_{ma'}C(O)CHR^{3a'}C(O)R^{2a'}$, wobei $R^{1a'}$, $R^{2a'}$, $R^{3a'}$ und $m^{a'}$ die oben angegebenen Bedeutungen haben, im molaren Verhältnis von höchstens 1 : 2 umsetzt.

7. Verfahren zur Herstellung von Dichlorobis(benzoylacetonato) hafnium(IV), dadurch gekennzeichnet, daß man Hafniumtetrachlorid mit Benzoylaceton unter Feuchtigkeitsausschluß umsetzt.

8. Verfahren zur Herstellung von Dibromobis(benzoylacetonato) zirkon(IV), dadurch gekennzeichnet, daß man Zirkontetrabromid mit Benzoylaceton unter Feuchtigkeitsausschluß umsetzt.

9. Verfahren zur Herstellung von Kopräzipitaten von Komplexverbindungen der allgemeinen Formel I

$$[R^1(CH_2)_mC(O)CR^3C(O)R^2]_{2+n}MX_{2-n} \quad (I),$$

worin

M Zinn, Titan, Zirkon oder Hafnium,

$R^1$ Wasserstoff oder einen Phenylrest, wobei der Phenylrest durch Fluor, Chlor, Brom, Nitro, $C_1$-$C_4$-Alkyl, $C_1$-$C_4$-Alkoxy oder Trifluormethyl einfach oder mehrfach substituiert sein kann,

$R^2$ Methyl oder $R^1$ mit Ausnahme von Wasserstoff,

$R^3$ Wasserstoff oder Chlor,

X Fluor, Chlor oder Brom, jedoch nicht Fluor, wenn M die Bedeutung Zirkon oder Hafnium hat,

m die Zahl 1 oder 0 oder, falls $R^1$ die Bedeutung Wasserstoff hat, die Zahl 1, und

n die Zahl 0 oder, falls M die Bedeutung Zirkon oder Hafnium hat, die Zahl 1 oder 0, bedeuten, und hydrophilen Polymeren, dadurch gekennzeichnet, daß man eine Lösung einer Komplexverbindung der allgemeinen Formel I und eines hydrophilen Polymeren in einem inerten organischen Lösungsmittel bis zur Trockne einengt.

10. Verfahren nach Anspruch 9, dadurch gekennzeichnet, daß man Polyvinylpyrrolidon als hydrophiles Polymer einsetzt.

11. Verfahren nach Anspruch 9, dadurch gekennzeichnet, daß es sich bei den hydrophilen Polymeren um Polyoxyethylensorbitanmonofettsäureester handelt.

12. Verfahren nach Anspruch 11, dadurch gekennzeichnet, daß es sich bei dem verwendeten Polyoxyethylensorbitanmonofettsäureester um Polyoxyethylen (20) sorbitanmonostearat handelt.

13. Verfahren nach Anspruch 11, dadurch gekennzeichnet, daß es sich bei dem verwendeten

Polyoxyethylensorbitanmonofettsäureester um Polyoxyethylen (20) sorbitanmonooleat handelt.

14. Verfahren zur Herstellung eines Kopräzipitats aus Dichlorobis(benzoylacetonato) titan(IV) und Polyoxyethylen (20) sorbitanmonostearat, dadurch gekennzeichnet, daß man eine Lösung der beiden Ausgangsstoffe in einem inerten organischen Lösungsmittel zur Trockne eingengt.

15. Verfahren zur Herstellung eines Kopräzipitats aus Dichlorobis(benzoylacetonato) titan(IV) und Polyoxyethylen (20) sorbitanmonooleat, dadurch gekennzeichnet, daß man eine Lösung der beiden Ausgangsstoffe in einem inerten organischen Lösungsmittel zur Trockne eingengt.

**Claims** (for the Contracting States : BE, CH, DE, FR, GB, IT, LI, LU, NL, SE)

1. Complex compounds of the general formula I

$$[R^1(CH_2)_mC(O)CR^3C(O)R^2]_{2+n}MX_{2-n} \qquad (I)$$

wherein

M denotes tin, titanium, zirconium or hafnium,

$R^1$ denotes hydrogen or a phenyl radical, it being possible for the phenyl radical to be monosubstituted or polysubstituted by fluorine, chlorine, bromine, nitro, $C_1$-$C_4$-alkyl, $C_1$-$C_4$-alkoxy or trifluoromethyl,

$R^2$ denotes methyl or $R^1$, with the exception of hydrogen,

$R^3$ denotes hydrogen or chlorine,

X denotes fluorine, chlorine or bromine, but not fluorine if M denotes zirconium or hafnium,

m denotes the number 1 or 0, or, if $R^1$ denotes hydrogen, the number 1 and

n denotes the number 0, or, if M denotes zirconium or hafnium, the number 1 or 0, for use in the treatment of cancers.

2. Complexes of the general formula I'

$$[R^{1'}(CH_2)_{m'}C(O)CR^{3'}C(O)R^{2'}]_{2+n'}M'X'_{2-n'} \qquad (I')$$

wherein

M' denotes tin, titanium, zirconium or hafnium,

$R^{1'}$ denotes a phenyl radical, which can be substituted by 4-chloro, 4-bromo, 3-nitro, 4-methoxy, 3,4-dimethoxy, 4-methyl or 2,4,6-trimethyl,

$R^{2'}$ denotes methyl or $R^{1'}$,

$R^{3'}$ denotes hydrogen or chlorine,

X' denotes fluorine, chlorine or bromine, but not fluorine if M' denotes zirconium or hafnium,

m' denotes the number 1 or 0 and

n' denotes the number 0, or, if M' denotes zirconium or hafnium, the number 1 or 0, for use in the treatment of cancers.

3. Complexes of the general formula I"

$$[R^{1''}(CH_2)_{m''}C(O)CR^{3''}C(O)R^{2''}]_{2+n''}M''X''_{2-n''} \qquad (I'')$$

wherein

M" denotes tin, titanium, zirconium or hafnium,

$R^{1''}$ denotes a phenyl radical,

$R^{2''}$ denotes a methyl radical,

$R^{3''}$ denotes hydrogen or chlorine,

X" denotes fluorine, chlorine or bromine, but not fluorine of M" denotes zirconium or hafnium, and

n" denotes the number 0, or, if M" denotes zirconium or hafnium, the number 1 or 0, for use in the treatment of cancers.

4. Dichlorobis(benzoylacetonato) titanium(IV), dichlorobis(benzoylacetonato) zirconium(IV) and/or dichlorobis(benzoylacetonato) hafnium(IV), for use in the treatment of cancers.

5. Compounds of the general formula $I^a$

$$[R^{1a}(CH_2)_{m^a}C(O)CR^{3a}C(O)R^{2a}]_{2+n^a}M^aX^a_{2-n^a} \qquad (I^a)$$

wherein

$M^a$ denotes tin, titanium, zirconium or hafnium,

$R^{1a}$ denotes a phenyl radical, which is monosubstituted or polysubstituted by fluorine, chlorine, bromine, nitro, $C_1$-$C_4$-alkyl, $C_1$-$C_4$-alkoxy or trifluoromethyl, and if $M^a$ represents zirconium or hafnium, additionally denotes a phenyl radical,

$R^{2a}$ denotes methyl or $R^{1a}$, including phenyl for all the meanings of $M^a$,

$R^{3a}$ denotes hydrogen or chlorine,

$X^a$ denotes fluorine, chlorine or bromine,

but not fluorine if $M^a$ denotes zirconium or hafnium, and not chlorine, if $M^a$ represents titanium or tin and $R^{1a}$ represents p-fluoro-, p-chloro- or p-bromo-phenyl and $R^{2a}$ represents methyl or if $M^a$ represents zirconium and $R^{1a}$ represents phenyl or if $M^a$ represents hafnium and $R^{1a}$ represents phenyl and $R^{2a}$ represents methyl and $n^a$ represents 1 or if $M^a$ represents tin and $R^{1a}$ and $R^{2a}$ represent phenyl, and not bromine if $M^a$ represents zirconium or hafnium and $R^{1a}$ and $R^{2a}$ represent phenyl or if $M^a$ represents tin and $R^{1a}$ represents phenyl or p-fluoro-phenyl and $R^{2a}$ represents methyl,

$m^a$ denotes the number 1 or 0 and

$n^a$ denotes the number 0, or, if $M^a$ denotes zirconium or hafnium, the number 1 or 0.

6. Compounds of the general formula $I^{a'}$

$$[R^{1a'}(CH_2)_{m^{a'}}C(O)CR^{3a'}C(O)R^{2a'}]_{2+n^{a'}}M^{a'}X^{a'}{}_{2-n^{a'}} \qquad (I^a)$$

wherein

$M^{a'}$ denotes zirconium or hafnium,

$R^{1a'}$ denotes phenyl,

$R^{2a'}$ denotes methyl,

$R^{3a'}$ denotes hydrogen or chlorine,

$X^{a'}$ denotes chlorine or bromine, but not chlorine if $M^{a'}$ represents zirconium or if $M^{a'}$ represents hafnium and $n^{a'}$ represents the number 1,

$m^{a'}$ denotes the number 1 or 0 and

$n^{a'}$ denotes the number 1 or 0.

7. Dichlorobis(benzoylacetonato) hafnium(IV).

8. Dibromobis(benzoylacetonato) zirconium(IV).

9. Process for the preparation of the compounds according to Claim 5 or 6, of the general formulae $I^a$ and $I^{a'}$, wherein $R^{1a}$ and $R^{1a'}$, $R^{2a}$ and $R^{2a'}$, $R^{3a}$ and $R^{3a'}$, $X^a$ and $X^{a'}$, $m^a$ and $m^{a'}$ and $n^a$ and $n^{a'}$ have the meanings given in Claim 5 or 6, characterized in that a metal tetrahalide $M^aX_4{}^a$ or $M^{a'}X_4{}^{a'}$, wherein $M^a$ and $M^{a'}$ and $X^a$ and $X^{a'}$ have the meanings given in Claim 5 or 6, is reacted with a diketone $R^{1a}(CH_2)_{m^a}C(O)CHR^{3a}C(O)R^{2a}$ or $R^{1a'}(CH_2)_{m^{a'}}C(O)CHR^{3a'}C(O)R^{2a'}$, wherein $R^{1a}$ and $R^{1a'}$, $R^{2a}$ and $R^{2a'}$, $R^{3a}$ and $R^{3a'}$ and $m^a$ and $m^{a'}$ have the meanings given in Claim 5 or 6, in the absence of moisture in an inert solvent in a molar ratio of at most 1 : 2.

10. Process according to Claim 9 for the preparation of dichlorobis(benzoylacetonato) hafnium(IV), characterized in that hafnium tetrachloride is reacted with benzoylacetone.

11. Process according to Claim 9 for the preparation of dibromobis(benzoylacetonato) zirconium(IV), characterized in that zirconium tetrabromide is reacted with benzoylacetone.

12. Medicaments containing one or more complexes according to one or more of Claims 5 to 8.

13. Compounds according to one or more of Claims 5 to 8 for use in the treatment of cancers.

14. Coprecipitates obtained from the complexes of the general formula I

$$[R^1(CH_2)_mC(O)CR^3C(O)R^2]_{2+n}MX_{2-n} \qquad (I)$$

wherein

M denotes tin, titanium, zirconium or hafnium,

$R^1$ denotes hydrogen or a phenyl radical, it being possible for the phenyl radical to be monosubstituted or polysubstituted by fluorine, chlorine, bromine, nitro, $C_1$-$C_4$-alkyl, $C_1$-$C_4$-alkoxy or trifluoromethyl,

$R^2$ denotes methyl or $R^1$, with the exception of hydrogen,

$R^3$ denotes hydrogen or chlorine,

X denotes fluorine, chlorine or bromine, but not fluorine if M denotes zirconium or hafnium,

m denotes the number 1 or 0, or, if $R^1$ denotes hydrogen, the number 1 and

n denotes the number 0, or, if M denotes zirconium or hafnium, the number 1 or 0,

and hydrophilic polymers.

15. Coprecipitates according to Claim 14, characterized in that the hydrophilic polymer is polyvinylpyrrolidone.

16. Coprecipitates according to Claim 14, characterized in that the hydrophilic polymer is a polyoxyethylene sorbitan fatty acid monoester.

17. Coprecipitates according to Claim 16, characterized in that the polyoxyethylene sorbitan fatty acid monoester is polyoxyethylene (20) sorbitan monostearate.

18. Coprecipitates according to Claim 16, characterized in that the polyoxyethylene sorbitan fatty acid monoester is polyoxymethylene (20) sorbitan monooleate.

19. Coprecipitate of dichlorobis(benzoylacetonato) titanium(IV) and polyoxyethylene (20) sorbitan monostearate.

20. Coprecipitate of dichlorobis(benzoylacetonato) titanium(IV) and polyoxyethylene (20) sorbitan monooleate.

21. Process for the preparation of coprecipitates according to one of Claims 14 to 20, characterized in that a solution of a complex of the general formula I

$$[R^1(CH_2)_mC(O)CR^3C(O)R^2]_{2+n}MX_{2-n} \qquad (I)$$

wherein

M denotes tin, titanium, zirconium or hafnium,

$R^1$ denotes hydrogen or a phenyl radical, it being possible for the phenyl radical to be monosubstituted or polysubstituted by fluorine, chlorine, bromine, nitro, $C_1$-$C_4$-alkyl, $C_1$-$C_4$-alkoxy or trifluoromethyl,

$R^2$ denotes methyl or $R^1$, with the exception of hydrogen,

$R^3$ denotes hydrogen or chlorine,

X denotes fluorine, chlorine or bromine, but not fluorine if M denotes zirconium or hafnium,

m denotes the number 1 or 0, or, if $R^1$ denotes hydrogen, the number 1 and

n denotes the number 0, or, if M denotes zirconium or hafnium, the number 1 or 0,

and of a hydrophilic polymer in an inert organic solvent is concentrated to dryness.

22. Sterile aqueous solutions containing coprecipitates according to one of Claims 14 to 20.

**Claims** (for the Contracting State AT)

1. Process for the preparation of agents having an antineoplastic action, characterised in that complex compounds of the general formula I

$$[R^1(CH_2)_mC(O)CR^3C(O)R^2]_{2+n}MX_{2-n} \qquad (I)$$

wherein

M denotes tin, titanium, zirconium or hafnium,

$R^1$ denotes hydrogen or a phenyl radical, it being possible for the phenyl radical to be monosubstituted or polysubstituted by fluorine, chlorine, bromine, nitro, $C_1$-$C_4$-alkyl, $C_1$-$C_4$-alkoxy or trifluoromethyl,

$R^2$ denotes methyl or $R^1$, with the exception of hydrogen,

$R^3$ denotes hydrogen or chlorine,

X denotes fluorine, chlorine or bromine, but not fluorine if M denotes zirconium or hafnium,

m denotes the number 1 or 0 or, if $R^1$ denotes hydrogen, the number 1 and

n denotes the number 0 or, if M denotes zirconium or hafnium, the number 1 or 0,

are prepared by reacting a metal tetrahalide $MX_4$, wherein M and X have the meanings given in the case of formula I, with a diketone $R^1(CH_2)_mC(O)CHR^3C(O)R^2$, wherein $R^1$, $R^2$, $R^3$ and m have the meanings given in formula I, in a molar ratio of not more than 1 : 2, with exclusion of moisture in an inert solvent, and in that one or more of the complex compounds of the formula I thus obtained are brought into a form suitable for administration as an agent having an antineoplastic action by mixing with a suitable pharmaceutically acceptable excipient.

2. Process for the preparation of agents having an antineoplastic action, characterised in that complex compounds of the general formula I'

$$[R^{1'}(CH_2)_{m'}C(O)CR^{3'}C(O)R^{2'}]_{2+n'}M'X'_{2-n'} \qquad (I')$$

wherein

M' denotes tin, titanium, zirconium or hafnium,

$R^{1'}$ denotes a phenyl radical, which can be substituted by 4-chloro, 4-bromo, 3-nitro, 4-methoxy, 3,4-dimethoxy, 4-methyl or 2,4,6-trimethyl,

$R^{2'}$ denotes methyl or $R^{1'}$,

$R^{3'}$ denotes hydrogen or chlorine,

X' denotes fluorine, chlorine or bromine, but not fluorine if M' denotes zirconium or hafnium,

m' denotes the number 1 or 0 and

n' denotes the number 0 or , if M' denotes zirconium or hafnium, the number 1 or 0,

are prepared by reacting a metal tetrahalide $M'X'_4$, wherein M' and X' have the meanings given in the case of formula I', with a diketone $R^{1'}(CH_2)_{m'}C(O)CHR^{3'}C(O)R^{2'}$, wherein $R^{1'}$, $R^{2'}$, $R^{3'}$ and m' have the meanings given in the case of formula I', in a molar ratio of not more than 1 : 2, with exclusion of moisture in an inert solvent, and in that one or more of the complex compounds of the formula I' thus obtained are brought into a form suitable for administration as an agent having an antineoplastic action by mixing with a suitable pharmaceutically acceptable excipient.

3. Process for the preparation of agents having an antineoplastic action, characterised in that complex compounds of the general formula I"

$$[R^{1''}C(O)CR^{3''}C(O)R^{2''}]_{2+n''}M''X''_{2-n''} \qquad (I'')$$

wherein

M" denotes tin, titanium, zirconium or hafnium,

$R^{1''}$ denotes a phenyl radical,
$R^{2''}$ denotes a methyl radical,
$R^{3''}$ denotes hydrogen or chlorine,
X" denotes fluorine, chlorine or bromine, but not fluorine if M" denotes zirconium or hafnium,
n" denotes the number 0 or, if M" denotes zirconium or hafnium, the number 1 or 0,
are prepared by reacting a metal tetrahalide $M''X''_4$, wherein M" and X" have the meanings given in the case of formula I", with a diketone $R^{1''}C(O)CHR^{3''}C(O)R^{2''}$ wherein $R^{1''}$, $R^{2''}$ and $R^{3''}$ have the meanings given in the case of formula I", in a molar ratio of not more than 1 : 2, with exclusion of moisture in an inert solvent, and in that one or more of the complex compounds of the formula I" thus obtained are brought into a form suitable for administration as an agent having an antineoplastic action by mixing with a suitable pharmaceutically acceptable excipient.

4. Process for the preparation of agents having an antineoplastic action, characterised in that the complex compound dichlorobis(benzoylacetonato) titanium(IV) is prepared by reacting titanium (IV) tetrachloride with benzoylacetone in a molar ratio of not more than 1 : 2, with exclusion of moisture in an inert solvent, and in that the dichlorobis(benzoylacetonato) titanium(IV) is brought into a form suitable for administration as an agent having an antineoplastic action by mixing with a suitable pharmaceutically acceptable excipient.

5. Process for the preparation of compounds of the general formula $I^a$

$$[R^{1a}(CH_2)_{m^a}C(O)CR^{3a}C(O)R^{3a}]_{2+n^a}M^aX^a_{2-n^a} \qquad (I^a)$$

wherein
$M^a$ denotes tin, titanium, zirconium or hafnium,
$R^{1a}$ denotes a phenyl radical which is monosubstituted or polysubstituted by fluorine, chlorine, bromine, nitro, $C_1$-$C_4$-alkyl, $C_1$-$C_4$-alkoxy or trifluoromethyl, or, if $M^a$ represents zirconium of hafnium, additionally a phenyl radical,
$R^{2a}$ denotes methyl or $R^{1a}$, including phenyl for all the meanings of $M^a$,
$R^{3a}$ denotes hydrogen or chlorine,
$X^a$ denotes fluorine, chlorine or bromine,
but not fluorine if $M^a$ represents zirconium of hafnium and not chlorine if $M^a$ represents titanium or tin and $R^{1a}$ represents p-fluoro-, p-chloro- or p-bromo-phenyl and $R^{2a}$ represents methyl or if $M^a$ represents zirconium and $R^{1a}$ represents phenyl or if $M^a$ represents hafnium and $R_{1a}$ represents phenyl and $R^{2a}$ represents methyl and $n^a$ represents 1 or if $M^a$ represents Sn and $R^{1a}$ and $R^{2a}$ represent phenyl, and not bromine if $M^a$ represents zirconium or hafnium and $R^{1a}$ and $R^{2a}$ represent phenyl, or if $M^a$ represents tin and $R^{1a}$ represents phenyl or p-fluorophenyl and $R^{2a}$ represents methyl,
$m^a$ denotes the number 1 or 0 and
$n^a$ denotes the number 0 or, if $M^a$ denotes zirconium or hafnium, the number 1 or 0, which is characterised in that a metal halide $M^aX^a_4$, wherein wherein (sic) $M^a$ and $X^a$ have the abovementioned meanings, is reacted with a diketone $R^{1a}(CH_2)_{ma}C(O)CHR^{3a}C(O)R^{2a}$, wherein $R^{1a}$, $R^{2a}$, $R^{3a}$ and $m^a$ have the abovementioned meanings, in a molar ratio of not more than 1 : 2, with exclusion of moisture in an inert solvent.

6. Process for the preparation of compounds of the general formula $I^{a'}$

$$[R^{1a'}(CH_2)_{m^{a'}}C(O)CR^{3a'}C(O)R^{2a'}]_{2+n^{a'}}M^{a'}X^{a'}_{2-n^{a'}} \qquad (I^{a'})$$

wherein
$M^{a'}$ denotes zirconium or hafnium,
$R^{1a'}$ denotes phenyl,
$R^{2a'}$ denotes methyl,
$R^{3a'}$ denotes hydrogen or chlorine,
$X^{a'}$ denotes chlorine or bromine, but not chlorine if $M^{a'}$ represents zirconium or $M^{a'}$ represents hafnium and $n^{a'}$ represents the number 1,
$m^{a'}$ denotes the number 1 or 0 and
$n^{a'}$ denotes the number 0 or, if $M^{a'}$ denotes zirconium or hafnium, the number 1 or 0,
which is characterised in that a metal halide $M^{a'}X^{a'}_4$, wherein wherein (sic) $M^{a'}$ and $X^{a'}$ have the abovementioned meanings, is reacted with a diketone $R^{1a'}(CH_2)_{ma'}C(O)CHR^{3a'}C(O)R^{2a'}$, wherein $R^{1a'}$, $R^{2a'}$, $R^{3a'}$ and $m^{a'}$ have the abovementioned meanings, in a molar ratio of not more than 1 : 2, with exclusion of moisture in an inert solvent.

7. Process for the preparation of dichlorobis-(benzoylacetonato) hafnium (IV), characterised in that hafnium tetrachloride is reacted with benzoylacetone with exclusion of moisture.

8. Process for the preparation of dibromobis-(benzoylacetonato) zirconium(IV), characterised in that zirconium tetrabromide is reacted with benzoylacetone with exclusion of moisture.

9. Process for the preparation of coprecipitates of complex compounds of the general formula I

$$[R^1(CH_2)_mC(O)CR^3C(O)R^2]_{2+n}MX_{2-n} \qquad (I)$$

wherein

M denotes tin, titanium, zirconium or hafnium,

$R^1$ denotes hydrogen or a phenyl radical, it being possible for the phenyl radical to be monosubstituted or polysubstituted by fluorine, chlorine, bromine, nitro, $C_1$-$C_4$-alkyl, $C_1$-$C_4$-alkoxy or trifluoromethyl,

$R^2$ denotes methyl or $R^1$, with the exception of hydrogen,

$R^3$ denotes hydrogen or chlorine,

X denotes fluorine, chlorine or bromine, but not fluorine if M denotes zirconium or hafnium,

m denotes the number 1 or 0 or, if $R^1$ denotes hydrogen, the number 1 and

n denotes the number 0 or, if M denotes zirconium or hafnium, the number 1 or 0,

and hydrophilic polymers, characterised in that a solution of a complex compound of the general formula I and of a hydrophilic polymer in an inert organic solvent is concentrated to dryness.

10. Process according to Claim 9, characterised in that polyvinylpyrrolidone is employed as the hydrophilic polymer.

11. Process according to Claim 9, characterised in that the hydrophilic polymers are polyoxyethylenesorbitan mono-fatty acid esters.

12. Process according to Claim 11, characterised in that the polyoxyethylenesorbitan mono-fatty acid ester used is polyoxyethylene (20) sorbitan monostearate.

13. Process according to Claim 11, characterised in that the polyoxyethylene (20) sorbitan monooleate.

14. Process for the preparation of a coprecipitate of dichlorobis(benzoylacetonato)titanium (IV) and polyoxyethylene (20) sorbitan monostearate, characterised in that a solution of the two starting substances in an inert organic solvent is concentrated to dryness.

15. Process for the preparation of a coprecipitate of dichlorobis(benzoylacetonato)titanium (IV) and polyoxyethylene (20) sorbitan monooleate, characterised in that a solution of the two starting substances in an inert organic solvent is concentrated to dryness.

**Revendications** (pour les Etats contractants : BE, CH, DE, FR, GB, IT, LI, LU, NL, SE)

1. Composés complexes de formule générale I,

$$[R^1(CH_2)_mC(O)CR^3C(O)R^2]_{2+n}MX_{2-n} \quad (I)$$

dans laquelle,

M représente l'étain, le titane, le zirconium, ou le hafnium,

$R^1$ représente l'hydrogène ou un reste phényle, le reste phényle pouvant être mono- ou polysubstitué par du fluor, du chlore, du brome, un groupe nitro, un groupe alkyle en $C_1$-$C_4$, un groupe alcoxy en $C_1$-$C_4$ ou un groupe trifluorométhyle,

$R^2$ représente un groupe méthyle ou $R^1$, à l'exception de l'hydrogène,

$R^3$ représente de l'hydrogène ou le chlore,

X représente du fluor, du chlore ou du brome, mais toutefois pas le fluor si M représente le zirconium ou le hafnium,

m représente le nombre 1 ou 0 ou, si $R^1$ représente de l'hydrogène, le nombre 1, et

n représente le nombre 0 ou, si M représente le zirconium ou le hafnium, le nombre 1 ou 0, pour l'utilisation pour le traitement de carcinoses.

2. Composés complexes de formule générale I' :

$$[R^{1'}(CH_2)_{m'}C(O)CR^{3'}C(O)R^{2'}]_{2+n'}M'X'_{2-n'} \quad (I')$$

dans laquelle,

M' représente l'étain, le titane, le zirconium ou le hafnium,

$R^{1'}$ représente un reste phényle, qui peut être 4-chloro-, 4-bromo-, 3-nitro-, 4-méthoxy, 3,4-diméthoxy-, 4-méthyl- ou 2,4,6-triméthylsubstitué,

$R^{2'}$ représente un groupe méthyle ou $R^{1'}$,

$R^{3'}$ représente l'hydrogène ou le chlore

X' représente le fluor, le chlore ou le brome, mais toutefois pas le fluor si M' représente le zirconium ou le hafnium,

m' représente le nombre 1 ou 0,

n' représente le nombre 0 ou, si M' représente le zirconium ou le hafnium, le nombre 1 ou 0, pour l'utilisation pour le traitement des carcinoses.

3. Composés complexes de formule générale I'',

$$[R^{1''}C(O)CR^{3''}C(O)R^{2''}]_{2+n''}d1'M''X''_{2-n''} \quad (I''),$$

dans laquelle,

M'' représente l'étain, le titane, le zirconium ou le hafnium,

$R^{1''}$ représente un reste phényle,

$R^{2''}$ représente un reste méthyle,

$R^{3''}$ représente de l'hydrogène ou du chlore,

X'' représente le fluor, le chlore ou le brome, mais toutefois pas le fluor si M'' représente le zirconium ou le hafnium, et

n'' représente le nombre 0 ou, si M'' représente le zirconium ou le hafnium, le nombre 1 ou 0, pour l'utilisation pour le traitement des carcinoses.

4. Le dichlorobis(benzoylacétonato) titane (IV), le dichlorobis(benzoylacétonato) zirconium (IV) et/ou le dichlorobis(benzoylacétonato) hafnium (IV) pour l'utilisation pour le traitement des carcinoses.

5. Composés de formule générale $I^a$,

$$[R^{1a}(CH_2)_{m^a}C(O)CR^{3a}C(O)R^{2a}]_{2+n^a}M^aX^a_{2-n^a} \qquad (I^a)$$

dans laquelle,

$M^a$ représente l'étain, le titane, le zirconium, le hafnium,

$R^{1a}$ représente un reste phényle mono ou polysubstitué par du fluor, du chlore, du brome, un ou des groupes nitro, alkyle en $C_1$-$C_4$, alcoxy en $C_1$-$C_4$ ou trifluorométhyle, et en plus un reste phényle si $M^a$ représente du zirconium et du hafnium,

$R^{2a}$ représente un groupe méthyle ou $R^{1a}$, y compris un groupe phényle, quel que soit le sens de $M^a$,

$R^{3a}$ représente de l'hydrogène ou du chlore,

$X^a$ représente du fluor, du chlore ou du brome,

mais toutefois pas le fluor si $M^a$ représente du zirconium ou du hafnium, et $X^a$ ne représente pas du chlore, si $M^a$ représente le titane ou l'étain et $R^{1a}$ représente le reste p-fluorophényle, p-chlorophényle ou p-bromophényle et $R^{2a}$ représente le reste méthyle ou $M^a$ représente le zirconium et $R^{1a}$ un groupe phényle ou si $M^a$ représente du hafnium et $R^{1a}$ représente un groupe phényle et $R^{2a}$ représente un groupe méthyle et $n^a$ est égale à 1 ou si $M^a$ représente Sn et $R^{1a}$ et $R^{2a}$ représentent des groupes phényle, et $X^a$ ne représente pas du brome, si $M^a$ représente le zirconium ou le hafnium et $R^{1a}$ et $R^{2a}$ représentent des groupes phényle, ou si $M^a$ représente l'étain et $R^{1a}$ représente un groupe phényle ou p-fluorophényle, et $R^{2a}$ représente un groupe méthyle,

$m^a$ représente le nombre 1 ou 0, et

$n^a$ représente le nombre 0 ou, si $M^a$ représente le zirconium ou le hafnium, le nombre 1 ou 0.

6. Composés de formule générale $I^{a'}$,

$$[R^{1a'}(CH_2)_{m^{a'}}C(O)CR^{3a'}C(O)R^{2a'}]_{2+n^{a'}}M^{a'}X^{a'}_{2-n^{a'}} \qquad (I^{a'})$$

dans laquelle,

$M^{a'}$ représente le zirconium ou le hafnium,

$R^{1a'}$ représente un groupe phényle,

$R^{2a'}$ représente un groupe méthyle,

$R^{3a'}$ représente de l'hydrogène ou du chlore,

$X^{a'}$ représente du chlore ou du brome, mais pas le chlore, si $M^{a'}$ représente du zirconium ou si $M^{a'}$ représente du hafnium et $n^{a'}$ le nombre 1,

$m^{a'}$ représente le nombre 1 ou 0,

$n^{a'}$ représente le nombre 1 ou 0.

7. Le dichlorobis(benzoylacétonato) hafnium (IV).

8. Le dibromobis(benzoylacétonato) zirconium (IV).

9. Procédé de préparation des composés suivant la revendication 5 ou 6 de formule générale $I^a$ ou $I^{a'}$ dans lesquels, $R^{1a}$ ou $R^{1a'}$ ; $R^{2a}$ ou $R^{2a'}$ ; $R^{3a}$ ou $R^{3a'}$ ; $X^a$ ou $X^{a'}$, $m^a$ ou $m^{a'}$ et $n^a$ ou $n^{a'}$ ont les significations indiquées dans la revendication 5 ou 6, caractérisé par le fait que l'on fait réagir dans un rapport molaire de 1 : 2 au maximum un tétrahalogénure métallique $M^aX_4^a$ ou $M^{a'}X_4^{a'}$, dans lequel $M^a$ ou $M^{a'}$ et $X^a$ ou $X^{a'}$ ont les significations indiquées dans la revendication 5 ou 6, en l'absence d'humidité, dans un solvant inerte, avec une dicétone $R^{1a}(CH_2)_{m^a}C(O)CHR^{3a}C(O)R^{2a}$ ou $R^{1a'}(CH_2)_{m^{a'}}C(O)CHR^{3a'}C(O)R^{2a'}$, dans lequelles $R^{1a}$ ou $R^{1a'}$, $R^{2a}$ ou $R^{2a'}$ ; $R^{3a}$ ou $R^{3a'}$ et $m^a$ ou $m^{a'}$ ont les significations indiquées dans la revendication 5 ou 6.

10. Procédé suivant la revendication 9 de préparation de dichlorobis(benzoylacétonato) hafnium (IV), caractérisé par le fait que l'on fait réagir le tétrachlorure de hafnium avec la benzoylacétone.

11. Procédé suivant la revendication 9 de préparation de dibromobis(benzoylacétonato) zirconium (IV), caractérisé par le fait que l'on fait réagir le tétrabromure de zirconium avec la benzoylacétone.

12. Médicaments contenant un ou plusieurs composés complexes suivant l'une ou plusieurs des revendications 5 à 8.

13. Composés suivant une ou plusieurs des revendications 5 à 8 pour l'utilisation dans le traitement des carcinoses.

14. Coprécipités obtenus à partir des composés complexes de formule générale I,

$$[R^1(CH_2)_mC(O)CR^3C(O)R^2]_{2+n}MX_{2-n} \quad (I)$$

dans laquelle,

M représente l'étain, le titane, le zirconium ou le hafnium,

$R^1$ représente l'hydrogène ou un reste phényle, le reste phényle pouvant être mono- ou polysubstitué par du fluor, du chlore, du brome, un ou des groupes nitro, alkyle en $C_1$-$C_4$, alcoxy en $C_1$-$C_4$ ou trifluorométhyle,

$R^2$ représente un groupe méthyle ou $R^1$ à l'exception de l'hydrogène

$R^3$ représente de l'hydrogène ou du chlore,

X représente du fluor, du chlore ou du brome, mais toutefois pas le fluor si M représente le zirconium ou le hafnium,

m représente le nombre 1 ou 0 ou, si $R^1$ représente l'hydrogène, le nombre 1, et

n représente le nombre 0 ou, si M représente le zirconium ou le hafnium, le nombre 1 ou 0,

et de polymères hydrophiles.

15. Coprécipités suivant la revendication 14, caractérisés par le fait qu'il s'agit, pour les polymères hydrophiles, de polyvinylpyrrolidone.

16. Coprécipités suivant la revendication 14, caractérisés par le fait qu'il s'agit, pour les polymères hydrophiles, de monoesters d'acides gras et de polyoxyéthylène-sorbitan.

17. Coprécipités suivant la revendication 16, caractérisés par le fait que le monoester d'acide gras et de polyoxyéthylène-sorbitan est du monostéarate de polyoxyéthylène-sorbitan.

18. Coprécipité suivant la revendication 16, caractérisé par le fait que le monoester d'acide gras et de polyoxyéthylène-sorbitan est le monooléate de polyoxyéthylène (20)-sorbitan.

19. Coprécipité de dichlorobis(benzoylacétonato) titane (IV) et de monostéarate de polyoxyéthylène (20)-sorbitan.

20. Coprécipité de dichlorobis(benzoylacétonato) titane (IV) et de monooléate de polyoxyéthylène (20)-sorbitan.

21. Procédé de préparation de coprécipités suivant l'une des revendications 14 à 20, caractérisé par le fait qu'on concentre jusqu'à siccité, une solution d'un complexe de formule générale I,

$$[R^1(CH_2)_mC(O)CR^3C(O)R^2]_{2+n}MX_{2-n} \quad (I)$$

dans laquelle,

M représente l'étain, le titane, le zirconium ou le hafnium,

$R^1$ représente de l'hydrogène ou un reste phényle, le reste phényle pouvant être mono- ou polysubstitué par du fluor, du chlore, du brome, un ou des groupes nitro, alkyle en $C_1$-$C_4$, alcoxy en $C_1$-$C_4$ ou trifluorométhyle,

$R^2$ représente un groupe méthyle ou $R^1$ à l'exception de l'hydrogène,

$R^3$ représente l'hydrogène ou du chlore,

X représente du fluor, du chlore ou du brome, mais toutefois pas le fluor si M représente le zirconium ou le hafnium,

m représente le nombre 1 ou 0 ou, si $R^1$ représente l'hydrogène, le nombre 1, et

n représente le nombre 0 ou, si M représente le zirconium ou le hafnium, le nombre 1 ou 0, et d'un polymère hydrophile, dans un solvant organique inerte.

22. Solutions aqueuses stériles contenant des coprécipités suivant l'une des revendications 14 à 20.

**Revendications** (pour l'Etat contractant AT)

1. Procédé de préparation d'agents à activité antinéoplasique, caractérisé par le fait que l'on prépare des composés complexes de formule générale I,

$$[R^1(CH_2)_mC(O)CR^3C(O)R^2]_{2+n}MX_{2-n} \quad (I)$$

dans laquelle,

M représente l'étain, le titane, le zirconium ou le hafnium,

$R^1$ représente l'hydrogène ou un reste phényle, le reste phényle pouvant être mono- ou polysubstitué par du fluor, du chlore, du brome, un ou des groupes nitro, alkyle en $C_1$-$C_4$, alcoxy en $C_1$-$C_4$ ou trifluorométhyle

$R^2$ représente un groupe méthyle ou $R^1$ à l'exception de l'hydrogène,

$R^3$ représente de l'hydrogène ou du chlore,

X représente du fluor, du chlore ou du brome, mais toutefois pas le fluor si M représente le zirconium ou le hafnium,

m représente le nombre 1 ou 0 ou, si $R^1$ représente l'hydrogène, le nombre 1, et

n représente le nombre 0, ou si M représente le zirconium ou le hafnium, le nombre 1 ou 0,

en faisant réagir un tétrahalogénure métallique $MX_4$, où M et X ont les significations indiquées pour la formule I, en l'absence d'humidité, dans un solvant inerte, avec une dicétone

$R^1(CH_2)_mC(O)CHR^3C(O)R^2$, où $R^1$, $R^2$, $R^3$ et m ont les significations indiquées pour la formule I, dans un rapport molaire de 1 : 2 au maximum et en ce que un ou plusieurs des composés complexes de formule I ainsi obtenus sont mis, par mélange avec un support pharmaceutiquement acceptable, approprié, sous une forme appropriée à l'administration en tant qu'agent à activité antinéoplasique.

2. Procédé de préparation d'agents à activité antinéoplasique, caractérisé par le fait que l'on prépare des composés complexes de formule générale I',

$$[R^{1'}(CH_2)_{m'}C(O)CR^{3'}C(O)R^{2'}]_{2+n'}M'X'_{2-n'} \quad (I'),$$

dans laquelle,

M' représente l'étain, le titane, le zirconium, ou le hafnium,

$R^{1'}$ représente un reste phényle, qui peut être 4-chloro, 4-bromo, 3-nitro, 4-méthoxy, 3,4-diméthoxy, 4-méthyl, ou 2,4,6-triméthyl-substitué,

$R^{2'}$ représente un groupe méthyle ou $R^{1'}$,

$R^{3'}$ represente de l'hydrogène ou du chlore,

X' représente du fluor, du chlore, ou du brome, mais toutefois pas le fluor si M' représente le zirconium ou le hafnium,

m' représente le nombre 1 ou 0, et

n' représente le nombre 0 ou, si M' représente le zirconium ou le hafnium, le nombre 1 ou 0,

en faisant réagir un tétrahalogénure métallique $M'X'_4$, où M' et X' ont les significations indiquées pour la formule I', en l'absence d'humidité, dans un solvant inerte, avec une dicétone $R^{1'}(CH_2)_{m'}C(O)CHR^{3'}C(O)R^{2'}$, où $R^{1'}$, $R^{2'}$, $R^{3'}$ et m' ont les significations indiquées pour la formule I', dans un rapport molaire de 1 : 2 au maximum, et en ce que un ou plusieurs des composés complexes de formule I' ainsi obtenus sont mis, par mélange avec un support pharmaceutiquement acceptable approprié, sous une forme appropriée à l'administration comme agent à activité antinéoplasique.

3. Procédé de préparation d'agents à activité antinéoplasique, caractérisé par le fait que l'on prépare des composés complexes de formule générale I'',

$$[R^{1''}C(O)CR^{3''}C(O)R^{2''}]_{2+n''}M''X''_{2-n''} \quad (I''),$$

dans laquelle,

M'' représente l'étain, le titane, le zirconium ou le hafnium,

$R^{1''}$ représente un reste phényle,

$R^{2''}$ représente un reste méthyle,

$R^{3''}$ représente de l'hydrogène ou du chlore,

X'' représente le fluor, le chlore ou le brome, mais toutefois pas le fluor si M'' représente le zirconium ou le hafnium,

n'' représente le nombre 0 ou, si M'' représente le zirconium ou le hafnium, le nombre 1 ou 0,

en faisant réagir un tétrahalogénure métallique $M''X''_4$, où M'' et X'' ont les significations indiquées pour la formule I'', en l'absence d'humidité, dans un solvant inerte, avec une dicétone $R^{1''}C(O)CHR^{3''}C(O)R^{2''}$, où $R^{1''}$, $R^{2''}$ et $R^{3''}$ ont les significations indiquées pour la formule I'', dans un rapport molaire de 1 : 2 au maximum et en ce que un ou plusieurs des composés complexes de formule I'' ainsi obtenus, sont mis, par mélange avec un support pharmaceutiquement acceptable approprié, sous une forme appropriée à l'administration comme agent à activité antinéoplasique.

4. Procédé de préparation d'agents à activité antinéoplasique, caractérisé par le fait que l'on prépare le composé complexe dichlorobis(benzoylacétonato) titane (IV) en faisant réagir du tétrachlorure de titane (IV) en l'absence d'humidité, dans un solvant inerte, avec la benzoylacétone dans un rapport molaire 1 : 2 au maximum et en ce que le dichlorobis(benzoylacétonato) titane (IV) est amené, par mélange avec un support pharmaceutiquement acceptable approprié, sous une forme appropriée à l'administration comme agent à activité antinéoplasique.

5. Procédé de préparation de composés de formule générale $I^a$,

$$[R^{1a}(CH_2)_{ma}C(O)CR^{3a}C(O)R^{2a}]_{2+na}M^aX^a_{2-na} \quad (I^a),$$

dans laquelle,

$M^a$ représente l'étain, le titane, le zirconium ou le hafnium,

$R^{1a}$ représente un reste phényle mono- ou polysubstitué par du fluor, du chlore, du brome, un ou des groupes nitro, alkyle en $C_1$-$C_4$, alcoxy en $C_1$-$C_4$ ou trifluorométhyle, et en outre, un reste phényle si $M^a$ représente le zirconium ou le hafnium,

$R^{2a}$ représente un groupe méthyle ou $R^{1a}$, y compris un groupe phényle pour toutes les significations de $M^a$,

$R^{3a}$ représente de l'hydrogène ou du chlore,

$X^a$ représente le fluor, le chlore ou le brome,

mais toutefois pas le fluor si $M^a$ représente du zirconium ou du hafnium et pas le chlore si $M^a$ représente le titane ou l'étain et $R^{1a}$ représente un groupe p-fluorophényle, p-chlorophényle ou p-bromophényle et $R^{2a}$ représente un groupe méthyle ou si $M^a$ représente le zirconium et $R^{1a}$ représente un groupe phényle,

ou si $M^a$ représente le hafnium et $R^{1a}$ représente un groupe phényle et $R^{2a}$ un groupe méthyle et $n^a$ est égal à 1, ou si $M^a$ représente Sn et $R^{1a}$ et $R^{2a}$ représentent des groupes phényle, et $X^a$ ne représente pas le brome si $M^a$ représente le zirconium ou le hafnium et $R^{1a}$ et $R^{2a}$ des groupes phényle, ou si $M^a$ représente l'étain et $R^{1a}$ un groupe phényle ou p-fluorophényle, et $R^{2a}$ représente un groupe méthyle,

$m^a$ représente le nombre 1 ou 0, et

$n^a$ représente le nombre 0 ou, si $M^a$ représente le zirconium ou le hafnium, le nombre 1 ou 0,

qui est caractérisé par le fait qu'on fait réagir un halogénure métallique $M^aX^a_4$, où $M^a$ et $X^a$ ont les significations indiquées plus haut, en l'absence d'humidité, dans un solvant inerte, avec une dicétone $R^{1a}(CH_2)_{m^a}C(O)CHR^{3a}C(O)R^{2a}$, où $R^{1a}$, $R^{2a}$, $R^{3a}$ et $m^a$ ont les significations indiquées ci-dessus, dans un rapport molaire de 1 : 2 au maximum.

6. Procédé de préparation de composés de formule générale $I^{a'}$,

$$[R^{1a'}(CH_2)_{m^{a'}}C(O)CR^{3a'}C(O)R^{2a'}]_{2+n^{a'}}M^{a'}X^{a'}_{2-n^{a'}} \qquad (I^{a'})$$

dans laquelle,

$M^{a'}$ représente le zirconium ou le hafnium,

$R^{1a'}$ représente un groupe phényle,

$R^{2a'}$ représente un groupe méthyle,

$R^{3a'}$ représente de l'hydrogène ou du chlore,

$X^{a'}$ représente le chlore ou le brome, mais pas le chlore si $M^{a'}$ représente le zirconium, ou si $M^{a'}$ représente le hafnium et $n^{a'}$ le nombre 1,

$m^{a'}$ représente le nombre 1 ou 0, et

$n^{a'}$ représente le nombre 0 ou, si $M^{a'}$ représente le zirconium ou le hafnium, le nombre 1 ou 0,

qui est caractérisé par le fait que l'on fait réagir un halogénure métallique $M^{a'}X^{a'}_4$, où $M^{a'}$ et $X^{a'}$ ont les significations indiquées ci-dessus, en l'absence d'humidité, dans un solvant inerte, avec une dicétone $R^{1a'}(CH_2)_{m^{a'}}C(O)CHR^{3a'}C(O)R^{2a'}$, où $R^{1a'}$, $R^{2a'}$, $R^{3a'}$ ont les significations indiquées ci-dessus, dans un rapport molaire de 1 : 2 au maximum.

7. Procédé de préparation de dichlorobis(benzoylacétonato) hafnium (IV), caractérisé par le fait qu'on fait réagir le tétrachlorure de hafnium avec la benzoylacétone en l'absence d'humidité.

8. Procédé de préparation de dibromobis(benzoylacétonato) zirconium (IV), caractérisé par le fait qu'on fait réagir le tétrabromure de zirconium avec la benzoylacétone en l'absence d'humidité.

9. Procédé de préparation de coprécipités de composés complexes de formule générale I,

$$[R^1(CH_2)_mC(O)CR^3C(O)R^2]_{2+n}MX_{2-n} \qquad (I)$$

dans laquelle,

M représente l'étain, le titane, le zirconium ou le hafnium,

$R^1$ représente de l'hydrogène ou un reste phényle, le reste phényle pouvant être mono- ou polysubstitué par du fluor, du chlore, du brome, un ou des groupes nitro, alkyle en $C_1$-$C_4$, alcoxy en $C_1$-$C_4$ ou trifluorométhyle,

$R^2$ représente un groupe méthyle ou $R^1$, à l'exception de l'hydrogène,

$R^3$ représente de l'hydrogène ou du chlore,

X représente le fluor, le chlore ou le brome, mais toutefois pas le fluor si M représente le zirconium ou le hafnium,

m représente le nombre 1 ou 0 ou, si $R'$ représente l'hydrogène, le nombre 1, et

n représente le nombre 0 ou, si M représente le zirconium ou le hafnium, le nombre 1 ou 0,

et de polymères hydrophiles, caractérisé par le fait que l'on concentre jusqu'à siccité, une solution d'un composé complexe de formule générale I, et d'un polymère hydrophile, dans un solvant organique inerte.

10. Procédé suivant la revendication 9, caractérisé par le fait que l'on met en œuvre de la polyvinylpyrrolidone comme polymère hydrophile.

11. Procédé suivant la revendication 9, caractérisé par le fait qu'il s'agit, pour les polymères hydrophiles, de monoesters d'acides gras et de polyoxyéthylène-sorbitan.

12. Procédé suivant la revendication 11, caractérisé par le fait qu'il s'agit, pour le monoester d'acide gras et de polyoxyéthylène-sorbitan utilisé, de monostéarate de polyoxyéthylène (20)-sorbitan.

13. Procédé suivant la revendication 11, caractérisé par le fait qu'il s'agit, pour le monoester d'acide gras et de polyoxyéthylène-sorbitan utilisé, de monooléate de polyoxyéthylène (20)-sorbitan.

14. Procédé de préparation d'un coprécipité de dichlorobis(benzoylacétonato) titane (IV) et de monostéarate de polyoxyéthylène (20)-sorbitan, caractérisé par le fait que l'on concentre jusqu'à siccité une solution des deux matières premières dans un solvant organique inerte.

15. Procédé de préparation d'un coprécipité de dichlorobis(benzoylacétonato) titane (IV) et de monooléate de polyoxyéthylène (20)-sorbitan, caractérisé par le fait que l'on concentre jusqu'à siccité, une solution des deux matières premières dans un solvant organique inerte.